# EUROPEAN PATENT APPLICATION

(11) **EP 2 873 425 A1**
(43) Date of publication of application: **20.05.2015**
(21) Application number: 13193478.8
(22) Date of filing: 19.11.2013
(51) Int. Cl.: A61K 49/18, C07K 14/195

(54) **Production of magnetic nanoparticles in recombinant host cells**

(71) Applicant: Ludwig-Maximilians-Universität München, 80539 München (DE)
(72) Inventor: Schüler, Dirk, 82061 Neuried (DE); Kolinko, Isabel, 82152 Planegg (DE)
(74) Representative: Danner, Stefan

(57) **Abstract**

The present invention relates to a recombinant host cell, comprising in its genome preferably heterologous gene expression cassettes capable of being expressed in the host cell, the gene expression cassettes encoding a nucleic acid sequence encompassing at least 80% of the full-length sequence of the *mamAB* operon, the *mamGDFC* operon, and the tmms6 operon of a magnetotactic alphaproteobacterium, respectively, and preferably further comprising gene expression cassettes capable of being expressed in the host cell, the gene expression cassettes encoding a nucleic acid sequence encompassing at least 80% of the full-length sequence of the *mamXY* operon and/or the *feoAB1* operon of a magnetotactic alphaproteobacterium; wherein the recombinant host cell, upon expression of the gene expression cassettes in their entirety, is capable of producing magnetic nanoparticles. Preferably, the recombinant host cell is derived from *Rhodospirillum rubrum.* Furthermore, the present invention also relates to a corresponding method for producing such recombinant host cell by means of genetic transposition.

## Description

### FIELD OF THE INVENTION

The present invention relates to recombinant host cells being genetically engineered to allow the heterologous production of magnetic nanoparticles, preferably of magnetosomes derived from magnetotactic bacteria. More specifically, the recombinant host cells comprise in their genome the entire genetic information sufficient to express the biosynthetic pathway for the production of magnetic nanoparticles.

### BACKGROUND OF THE INVENTION

Magnetic nanoparticles typically comprise nanocrystals made of oxides (or to a lesser extent of sulfides) of the elements in the forth row of the periodic table (i.e. Cr, Mn, Fe, Co, and Ni). The ability to produce such magnetic nanoparticles is inevitable not only for the general understanding of magnetic properties in a nanometer regime, but also for manifold technical applications ranging from magnetic resonance imaging, drug delivery, catalysts, and biosensing to nanoelectronics, semiconductor materials, and magnetic storage media (reviewed in Lu, A. et al. (2007) Angew. Chem. Int. Ed. 46, 1222-1224).

Magnetic nanoparticles can be synthesized chemically through precipitation of the crystals from basic aqueous solutions. However, the production of particularly dimensioned ("tailored") nanocrystals via these synthesis routes is significantly hampered by the broad size distribution of the crystal populations obtained. More recently, the synthesis of nanocrystals has been directed to non-aqueous approaches generally resulting in the formation of crystals having not only an improved overall quality but also a narrower size distribution. Nevertheless, in most chemical syntheses reported so far only sub-gram to low gram quantities of monodisperse nanocrystals were obtained, not sufficient for many applications. Furthermore, only a fraction of such synthetic particles constitutes monocrystalline particles having defined magnetic properties. Typically, however, the resulting particles were too small for clinically relevant applications such as magneto-hyperthermic treatment of tumors (Park, J. et al. (2004) Nature Materials 3, 891-895).

Alternatively, biogenic magnetic nanoparticles can be employed that are produced by magnetotactic organisms, predominantly magnetotactic bacteria. The ability of magnetotactic bacteria to orient in the Earth's magnetic field is based on the presence of specific organelles, the magnetosomes, which are membrane-enveloped monocrystalline crystals of a magnetic mineral that are arranged in chain-like structures within the cell. Magnetosomes display a variety of species-specific shapes within the single magnetic domain size range (reviewed in Bazylinski, D.A. and Frankel, R.B. (2004) Nature Rev. Microbiol. 2, 217-230). In the prototypical *Magnetospirillum,* cubo-octahedral nanocrystals of the mineral magnetite (Fe₃O₄) having a maximal diameter of 50 nm are synthesized within magnetosome membrane (MM) vesicles. The MM is a phospholipid bilayer of a distinctive biochemical composition. Their specific structural and magnetic properties make these bacterial magnetosomes highly attractive for various nanotechnological and biomedical applications.

However, the biotechnological usability is hampered by the fact that they can only be produced in comparably small amounts due to the methodological difficulties to cultivate magnetotactic bacteria due to fastidious growth requirements. Typically, the cell yields obtained are only about 1 g (fresh weight) per liter of culture, not enough for the large scale production of magnetosomes (Heyen, U. and Schüler, D. (2003) Appl. Microbiol. Biotechnol. 61, 536-544). In addition, genetic interference in or manipulation of the genetic pathway for magnetosome production is cumbersome due to the limited availability of molecular tools for the recalcitrant native host cells.

The biogenesis of functional magnetosomes is highly complex and involves the invagination of magnetosome vesicles from the cytoplasmic membrane, the magnetosomal uptake of iron and the crystallization of magnetite particles, as well as their assembly into chains along a dedicated cytoskeletal structure (Komeili, A. et al. (2006) Science 311, 242-245; Katzmann, E. et al. (2010) Mol. Microbiol. 77, 208-224). Recently, in *M. gryphiswaldense,* genes controlling magnetosome synthesis within several clusters of a larger (115 kb) genomic magnetosome island (MAI) were discovered, which are interspersed by transposases and genes of unknown function (Jogler, C. et al. (2009) Environ. Microbiol. 11, 1267-1277; Jogler, C. et al. (2011) Proc. Natl. Acad. Sci. USA 108, 1134-1139). Whereas the smaller *mamGFDC* (2.1 kb), *mms6* (3.6 kb) and *mamXY* operons (5.1 kb) have accessory functions in biomineralization of properly sized and shaped crystals, the large *mamAB* operon (16.4 kb) encodes proteins essential for iron transport, magnetosome membrane formation, and crystallization of magnetosome particles as well as their assembly and intracellular positioning. However, due to the structural complexity of the magnetosome organelle, current knowledge about particular gene functions is still quite limited and the presumably complex interplay between the numerous factors involved in the production of magnetosomes needs still to be unraveled in closer detail which is further complicated by the limited methodology for genetic manipulation of magnetotactic bacteria that is presently available.

Thus, there is a need for molecular tools as well as corresponding methods in order to improve the yield of biogenic magnetic nanoparticles that can be obtained from bacterial cultures. In particular, there remains an ongoing need for techniques that allow for the large-scale production of magnetic nanoparticles in an easy-to-do and cost-efficient manner.

Accordingly, it is an object of the present invention to provide such molecular tools and corresponding methods.

### SUMMARY OF THE INVENTION

In one aspect, the present invention relates to a recombinant host cell, comprising in its genome: (i) a gene expression cassette capable of being expressed in the host cell, the gene expression cassette encoding a nucleic acid sequence encompassing at least 80% of the full-length sequence of the *mamAB* operon of a magnetotactic alphaproteobacterium; (ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium; and (iii) a gene expression cassette capable of being expressed in the host cell, the gene expression cassette encoding a nucleic acid sequence encompassing at least 80% of the full-length sequence of the *mms6* operon of a magnetotactic alphaproteobacterium; wherein the recombinant host cell, upon expression of the gene expression cassettes in their entirety, is capable of producing magnetic nanoparticles.

In preferred embodiments, the recombinant host cell further comprises: (iv) a gene expression cassette capable of being expressed in the host cell, the gene expression cassette encoding a nucleic acid sequence encompassing at least 80% of the full-length sequence of the *mamXY* operon of a magnetotactic alphaproteobacterium; and/or (v) a gene expression cassette capable of being expressed in the host cell, the gene expression cassette encoding a nucleic acid sequence encompassing at least 80% of the full-length sequence of the *feoAB1* operon of a magnetotactic alphaproteobacterium.

In specific embodiments, the recombinant host cell comprises the gene expression cassettes (i) to (v), wherein the gene expression cassettes all encompass the full-length sequences of the respective operons.

Particularly, the total length of the gene expression cassettes introduced in the recombinant host cell is less than 35 kb.

In further preferred embodiments, the magnetotactic alphaproteobacterium from which the respective operon sequences are derived is *Magnetospirillum spec.,* and in particular *Magnetospirillum gryphiswaldense.*

Preferably, the gene expression cassettes are stably integrated into the host cell's genome.

In specific embodiments, the magnetic nanoparticles are magnetosomes, and in particular magnetosomes consisting of magnetite.

In further specific embodiments, any one or more of the gene expression cassettes are under the control of their respective endogenous regulatory sequences.

In yet further specific embodiments, the recombinant host cell comprises two or more copies of any one or more of the gene expression cassettes.

Preferably, with respect to the recombinant host cell, any one or more of the gene expression cassettes represent heterologous nucleic acid sequences.

In further preferred embodiments, the recombinant host cell is a prokaryotic cell, and particularly derived from an alphaproteobacterium. Particularly preferably, the recombinant host cell is derived from *Rhodospirillum rubrum.*

In a further aspect, the present invention relates to a method for the production of a recombinant host cell as defined herein, the method comprising the transfer of the gene expression cassettes into the host cell by means of genetic transposition, and in particular comprising a modular transfer of the gene expression cassettes.

In yet another aspect, the present invention relates to the use of a recombinant host cell as defined herein for the biotechnological production of magnetic nanoparticles.

In yet another aspect, the present invention relates to the use of a recombinant host cell as defined herein for the *in vivo* synthesis of magnetic nanoparticles for application in magnetogenetics or biomedical imaging.

### BRIEF DESCRIPTION OF THE DRAWINGS

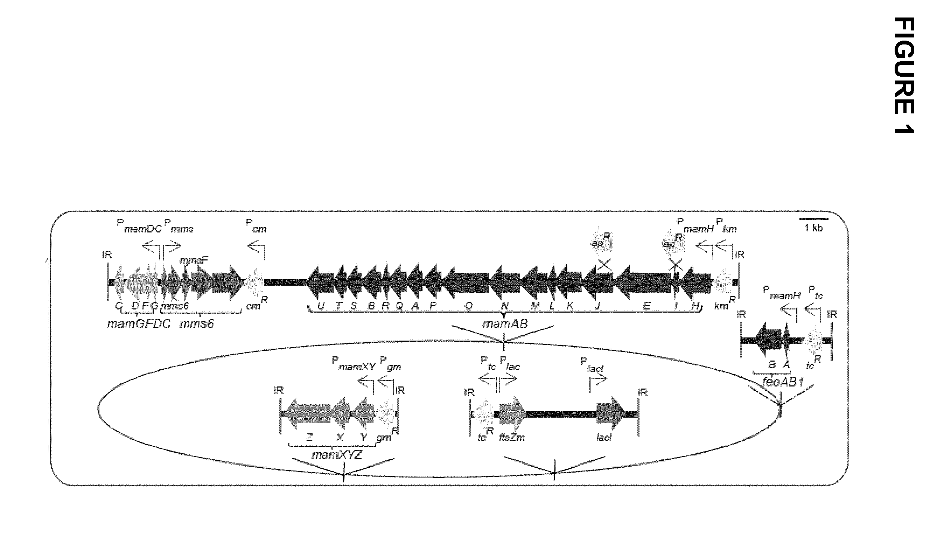

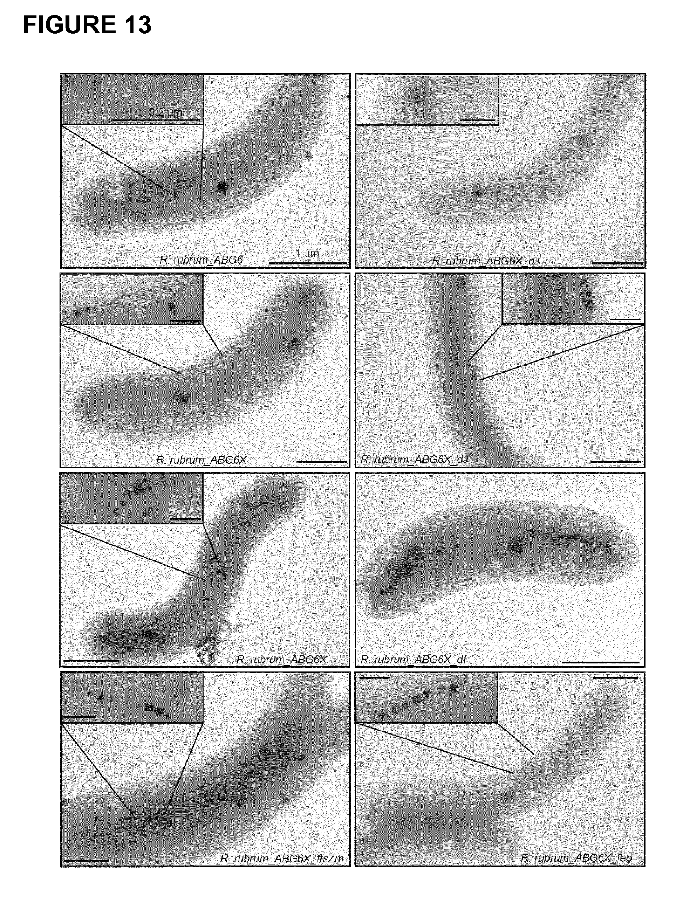

**FIGURE 1****: Schematic representation of *Magnetospirillum gryphiswaldense* gene clusters that were transferred to the chromosome of *Rhodospirillum rubrum.***
   Different colors indicate single genes or operons inserted into the chromosome (oval shape). Dark blue: *mamAB* operon, green: *mamGFDC* operon, brown: *mms6* operon, pale blue: *mamXY* operon, red: *feoAB1* operon, grey: *lacl* repressor gene, yellow: antibiotic resistance genes (*km^{R}*: kanamycin resistance, *tc^{R}*: tetracycline resistance, *ap^{R}*: ampicillin resistance, *gm^{R}*: gentamicin resistance). Red arrows indicate different promoters (P) controlling expression of antibiotic resistance genes (P*ₖₘ*, P*_{gm}*, P*_{tc}*), repressor genes (P_{*lac*/}) or magnetosome genes (P*ₘₘₛ*, P*_{mamDC}*, P*_{mamH}*, P*_{mamXY}*, *P_{lac}*). Crossed lines indicate gene deletions by homologous recombination that were introduced into the recipient genome to prove equivalent functions as in the donor organism. IR=inverted repeat defining the boundaries of the sequence inserted by the transposase.

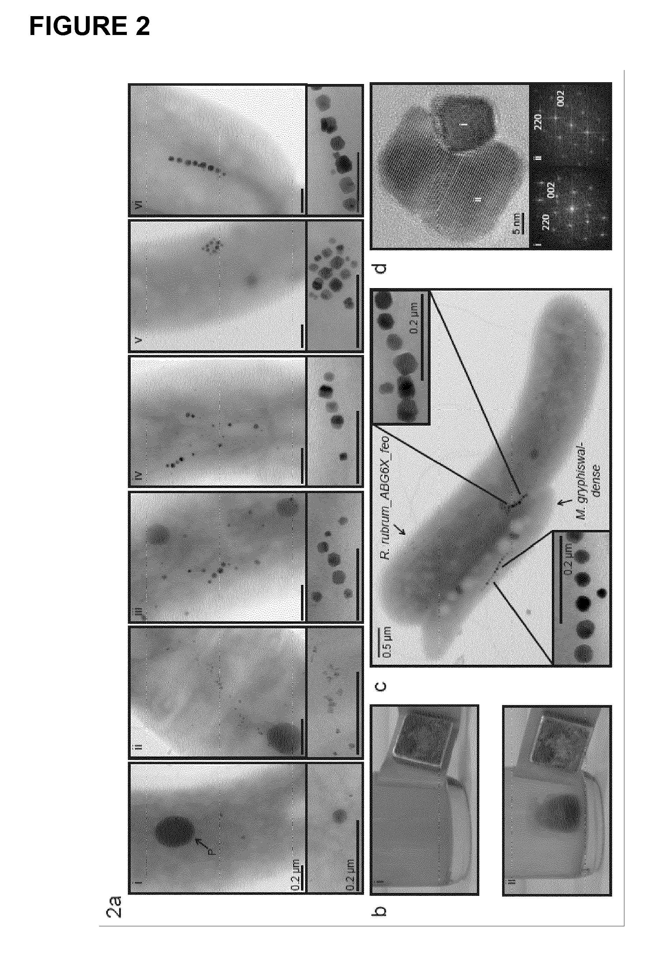

FIGURE 2: Phenotypes of *Rhodospirillum rubrum* strains expressing different magnetosome gene clusters and auxiliary genes.
   (2a) Transmission electron micrographs. *R. rubrum* wt (i) harbors larger phosphate inclusion (P) and some small, non-crystalline electron-dense particles. *R. rubrum_ABG6* (ii), *R. rubrum_ABG6X* (iii), *R. rubrum_ABG6X_ftsZm* (iv), *R. rubrum_ABG6X_dJ* (v), *R. rubrum_ABG6X_feo* (vi). Insets show magnifications of non-crystalline electron dense particles or heterologously expressed nanocrystals from (i)-(vi). (ii) *R. rubrum_ABG6* (*mamAB, mamGFDC,* and *mms6* operons inserted) showed small crystalline particles aligned in a chain-like pattern. (iii) Additional insertion of the *mamXY* operon lacking *ftsZm* resulted in formation of larger magnetic particles with an average size of 25 nm *(R. rubrum_ABG6X*). (iv) Additional transposition of *ftsZm* under control of an inducible *lac* promoter did not further increase crystal number and size. (v) Deletion of *mamJ* in *R. rubrum_ABG6X* caused agglomeration of the crystals into clusters. (vi) Additional insertion of *feoAB1* into *R. rubrum_ABG6X* resulted in increased magnetosome biomineralization (average crystal diameter 35 nm, formation of longer magnetosome chains of 440 nm). Scale bar: 0.2 µm. For further TEM micrographs see FIG. 13. **(2b)** Unlike the untransformed wild-type, cells of *R. rubrum_ABG6X* accumulated as a visible red spot near the pole of a permanent magnet at the edge of a culture flask. **(2c)** TEM micrograph of a mixed culture of the donor M. *gryphiswaldense* and the recipient *R. rubrum_ABG6X_feo*, illustrating characteristic cell properties and magnetosome organization. Insets show magnifications of magnetosomes from *M*. *gryphiswaldense* and *R. rubrum_ABG6X_feo.* **(2d)** HRTEM lattice image of a twinned crystal from *R. rubrum_ABG6X,* with the Fourier transforms (i) and (ii) that show intensity maxima consistent with the structures of magnetite, respectively.

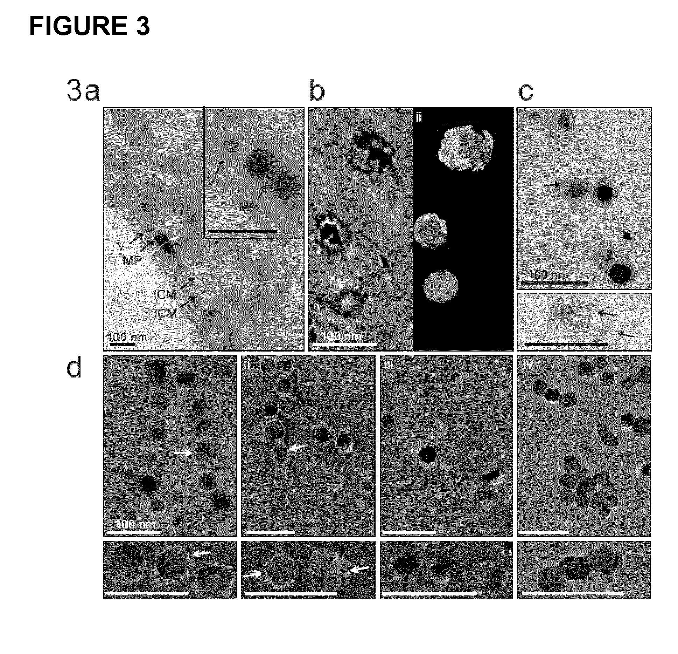

**FIGURE 3****: Ultrastructural analysis of *Rhodospirillum rubrum_*ABG6X and isolated crystals.**
   **(3a)** Cryo-fixed, thin-sectioned *R. rubrum_ABG6X* harbored intracytoplasmic membranes (ICMs) (93 ± 34 nm, n = 95), magnetic particles (MP) and a vesicle (V) enclosing an immature crystal (66 ± 6 nm, ± = s.d., n = 7.). The inset shows a magnification of the magnetite crystals. Scale bar: 100 nm. **(3b)** Cryo-electron tomography (CET) of isolated magnetic particles of *R. rubrum_ABG6X*: X-Y slice of a reconstructed tomogram (I) and surface-rendered 3D representation (II). A membrane-like structure (thickness 3.4 ± 1.0 nm, n = 6) surrounds magnetic particles (red). Yellow: membrane like structure, blue: empty vesicle. Scale bar: 100 nm. **(3c and 3d)** Transmission electron micrographs of isolated magnetosomes from *R. rubrum_ABG6X* (3c and 3d (ii), (iii), (iv)) and *M*. *gryphiswaldense* (3d (i)) negatively stained by **(3c)** uranyl acetate or **(3d)** phosphotungstic acid. Insets show magnifications of respective magnetic particles. Arrows indicate the magnetosome membrane, which encloses magnetic crystals of *M*. *gryphiswaldense* (3.2 ± 1.0 nm, n = 103) and *R. rubrum_ABG6X* (3.6 ± 1.2 nm, n = 100). Organic material could be solubilized from magnetite crystals of *R. rubrum_ABG6X* with SDS (iv) and less effective also by Triton X-100 (III). Scale bar: 100 nm.

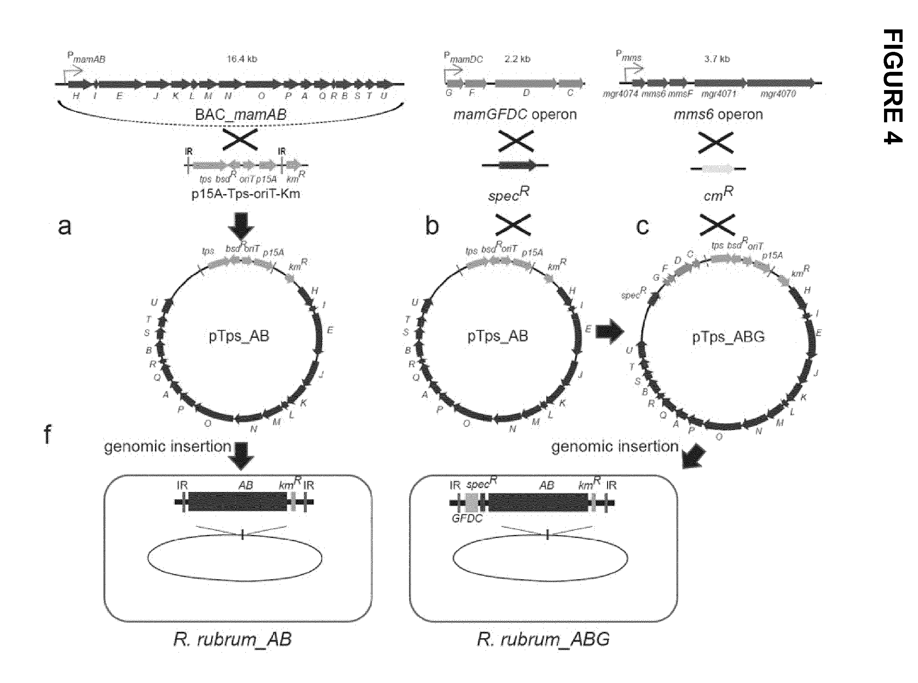

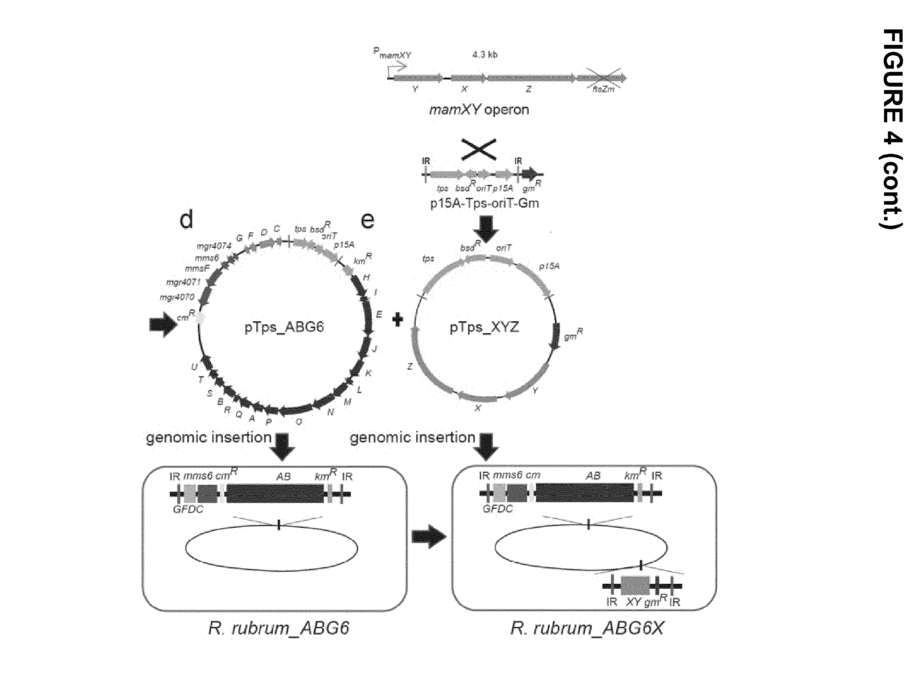

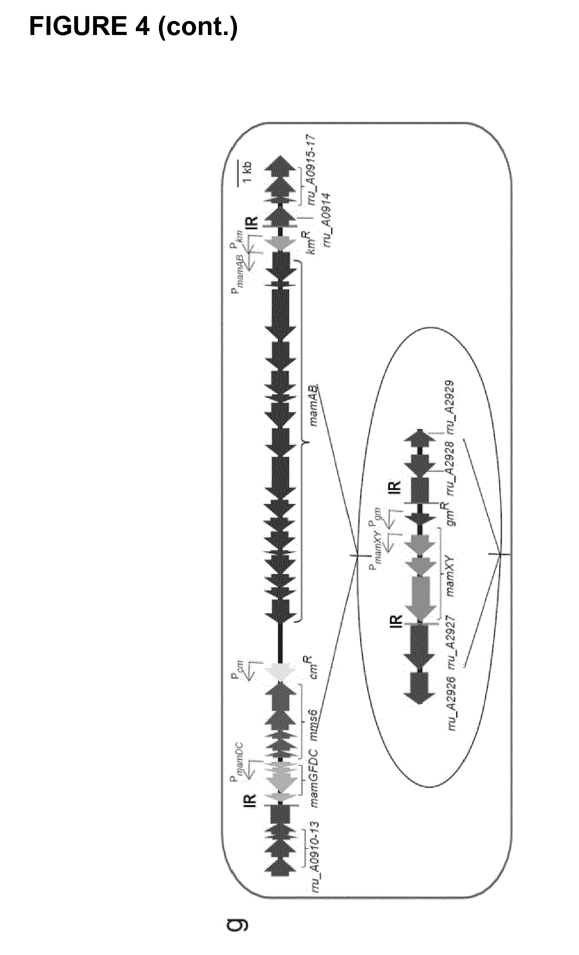

**FIGURE 4****: Construction scheme of insertion cassettes for modular expression of the *mam* and *mms* operons.**
   **(4a)** Recombinant engineering of a BAC containing the *mamAB* operon (blue) and a vector backbone (Km-p15A-Tps-oriT-Km, orange) harboring a MycoMar transposase gene (tps), inverted repeats (IR), origin of transfer (oriT), p15A origin of replication (p15A) and a *kanamycin^{R}* cassette (*km^{R},* orange). **(4b)** Insertion of a *spectinomycin^{R}* cassette (*spec^{R}*, pink) and the *mamGFDC* operon (green) into pTps_AB by triple recombination. **(4c and 4d)** Stitching of pTps_ABG by insertion of the *mms6* operon and a *chloramphenicol^{R}* cassette. **(4e)** pTps_XY consisting of a Tps vector backbone (orange), the *mamXY* operon (pale blue) and a *gentamicin^{R}* gene (*gm^{R}*, purple) was constructed. **(4f)** Plasmids were transferred by conjugation into *R. rubrum.* Transposition of the DNA fragments within the IR sequences occurred randomly at TA dinucleotide insertion sites by a "cut and paste" mechanism (Rubin, E.J. et al. (1990) Proc. Natl. Adac. Sci. USA 96, 1645-1650). **(4g)** Chromosomal insertion sites of the transposed constructs in *R. rubrum_ABG6X* are shown with adjacent genes (red) as revealed by whole genome sequencing performed with a MiSeq sequencer (Illumina) (accession number of *R. rubrum* ATCC 11170: NC_007643). pTps_ABG6 inserted within a gene encoding a putative aldehyd dehydrogenase (YP_426002), and pTps_XYZ inserted within rru_A2927, encoding an acriflavin resistance protein (protein accession number YP_428011). Sequences of inserted magnetosome operons matched those of the donor (M. *gryphiswaldense*) with no detectable mutations, except for a deletion (aa 169-247) within the hypervariable non-essential CAR domain of *mamJ,* which was shown to be irrelevant for protein function (Scheffel, A. and Schüler, D. (2007) J. Bacteriol. 189, 6437-6446).

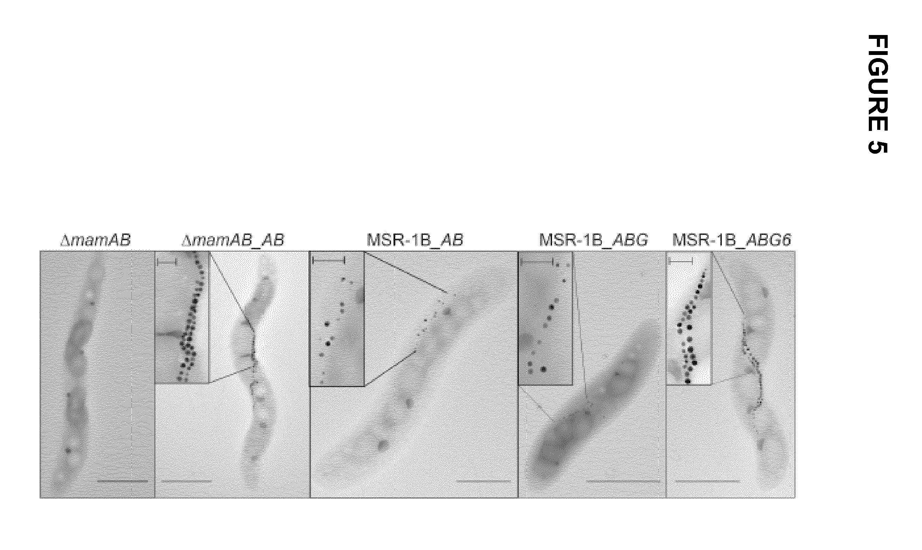

**FIGURE 5****: Transmission electron micrographs of MSR mutants expressing various insertional transposon constructs.**
   The plasmids pTps_AB, pTps_ABG and pTps_ABG6 were transferred into the non-magnetic *M. gryphiswaldense* mutants Δ*mamAB* and MSR-1 B, the latter lacking most of the magnetosome genes except of the *mamXY* operon (Lohsse, A. et al. (2011) PLoS One 6, e25561). After transfer of pTps_AB, a wt-like phenotype was restored in *ΔmamAB_AB* as revealed by C_{mag} (1.2 ± 0.2) and measured crystal sizes (37 ± 10 nm) in comparison with *M. gryphiswaldense* wild-type (36 ± 9 nm, C_{mag} = 1.4 ± 0.2) (see also Table 1). Mutant MSR-1 B was only partly complemented after insertion of pTps_AB and pTps_ABG, that is, C_{mag} and crystal sizes were still lower than in the wild-type (wt) (see also Table 1). Transfer of pTps_ABG6 restored nearly wild-type like magnetosome formation in MSR-1 B (35 ± 8 nm, C_{mag} = 0.9 ± 0.1). ± = s.d. Scale bar: 1 µm, insets: 0.2 µm.
**FIGURE 6****: HRTEM lattice image of a crystal from *Rhodospirillum rubrum_ABG6* with the corresponding Fourier transform (i) that shows intensity maxima consistent with the structures of hematite.**
**FIGURE 7****: Growth and magnetic response of *Rhodospirillum rubrum_AGB6X* cultivated under various growth conditions.**
   **(7a and 7b)** Cells were grown in ATCC 112 (chemotrophic, 20% O₂), Sistrom A (phototrophic, anoxygenic) and M2SF (chemotrophic, 1% O₂) medium for 3 (30°C), 4 (23°C) or 10 (18°C) days. Optical density at 660 and 880 nm and magnetic response were measured. The ratio OD₈₈₀/OD₆₆₀ correlates with the amount of chromatophores produced in the cells (median values n = 3, error bars indicate s.d.). No C_{mag} was detectable under aerobic and microaerobic conditions at 30°C. Anoxygenic and microaerobic conditions (23°C) supported particle formation. Furthermore, absorption spectra of extracted bacteriochlorophylls of *R. rubrum* WT **(7c)** and *R. rubrum_*ABG6X **(7d)** were directly measured, respectively (phototrophic growth, 30°C). No differences in absorption spectra were detectable.

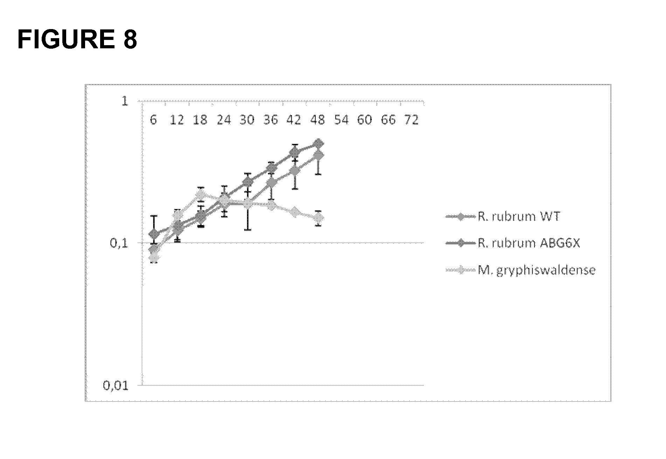

**FIGURE 8****: Growth of *Magnetospirillum gryphiswaldense* (OD₅₆₅), *Rhodospirillum rubrum* wt and *Rhodospirillum rubrum_ABG6X* (OD₆₆₀).**
   Cells of *M*. *gryphiswaldense* (FSM medium) or *R. rubrum* (Sistrom A medium, 1000 lux) were incubated for 3 days at 23°C under anaerobic conditions. Growth of the mutant strain *ABG6X* (median values n=3, error bars indicate s.d.) was indistinguishable from that of the untransformed wild-type (n=3).

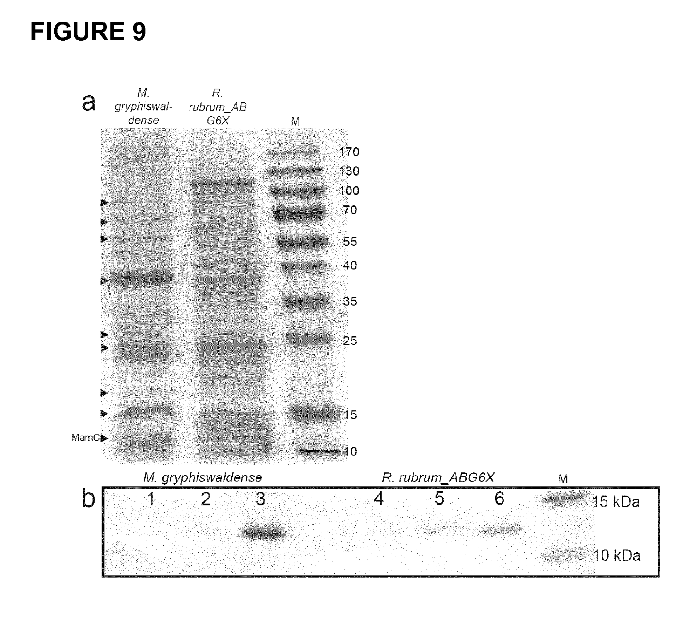

**FIGURE 9****: Proteomic analysis of magnetosomes from *Rhodospirillum rubrum_ABG6X.***
   (9a) 1 D-SDS-PAGE (Comassie blue stained) of proteins solubilized from isolated magnetosome particles of *M*. *gryphiswaldense* and *R. rubrum_ABG6X.* Bands (- 90, 70, 60, 40, 30, 25, 20, 15, 12.4 kDa) of the same size are indicated (arrowheads). **(9b)** Immunodetection of MamC (12.4 kDa) in blotted fractions of *M*. *gryphiswaldense* and *R. rubrum_ABG6X* using an anti-MamC antibody (Lang, C. and Schüler, D. (2008) Appl. Environ. Microbiol. 74, 4944-4953). A signal for MamC was detectable in the magnetic membrane fraction of *R. rubrum_ABG6X* (6), which was absent from the soluble fraction, but faintly present also in the non-magnetic membrane fraction (5), possibly originating from empty membrane vesicles or incomplete magnetic separation during isolation. Protein extracts from *M. gryphiswaldense*: 1. soluble fraction, 2. non-magnetic membrane fraction, 3. magnetosome membrane. Protein extracts from *R. rubrum_ABG6X*: 4. soluble fraction, 5. non-magnetic membrane fraction, 6. magnetic ("magnetosome") membrane fraction. M: Marker.

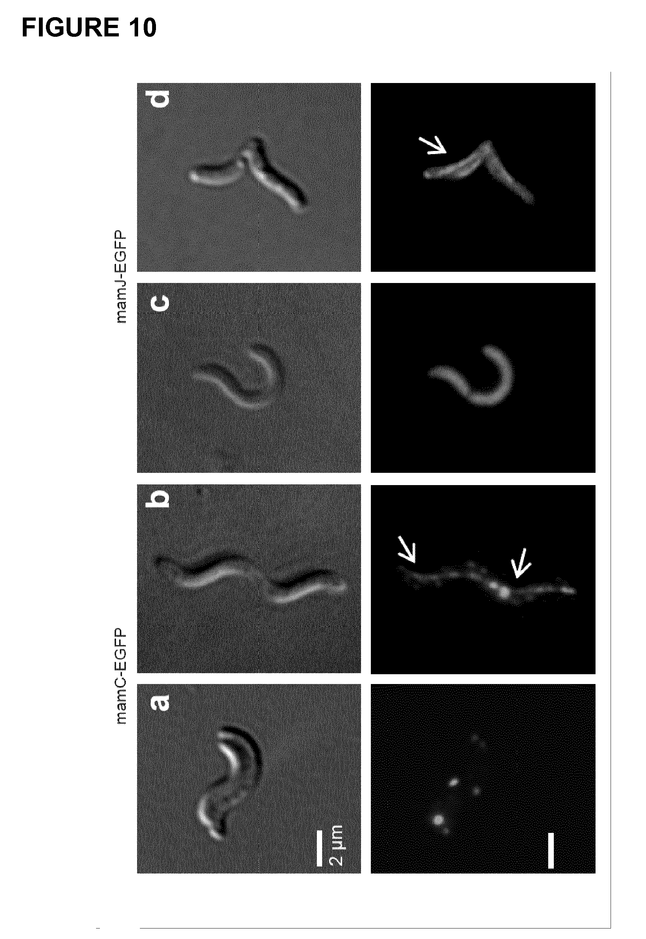

**FIGURE 10****: Fluorescence microscopy of *Rhodospirillum rubrum* WT and *Rhodospirillum rubrum_ABG6X* expressing different EGFP-tagged magnetosome proteins.**
   For localization studies of fluorescently labeled magnetosome proteins, strains were cultivated in ATCC medium overnight at 30 °C with appropriate antibiotics (Table S3). **(10a and 10b)** MamGFDC with a C-terminal MamC-EGFP fusion expressed in *R. rubrum* wt (n = 151) **(10a),** and *R. rubrum_ABG6X* (n = 112) **(10b).** In the transformed strain, a filamentous structure is visible for 79% of the cells (n = 89). **(10c and 10d)** MamJ-EGFP expressed in *R. rubrum* wt (n = 109) **(10c),** and in *R. rubrum_ABG6X* (n = 89) displaying a chain-like fluorescence signal in 63% of the cells (n = 56) **(10d).** Scale bar: 2 µm.

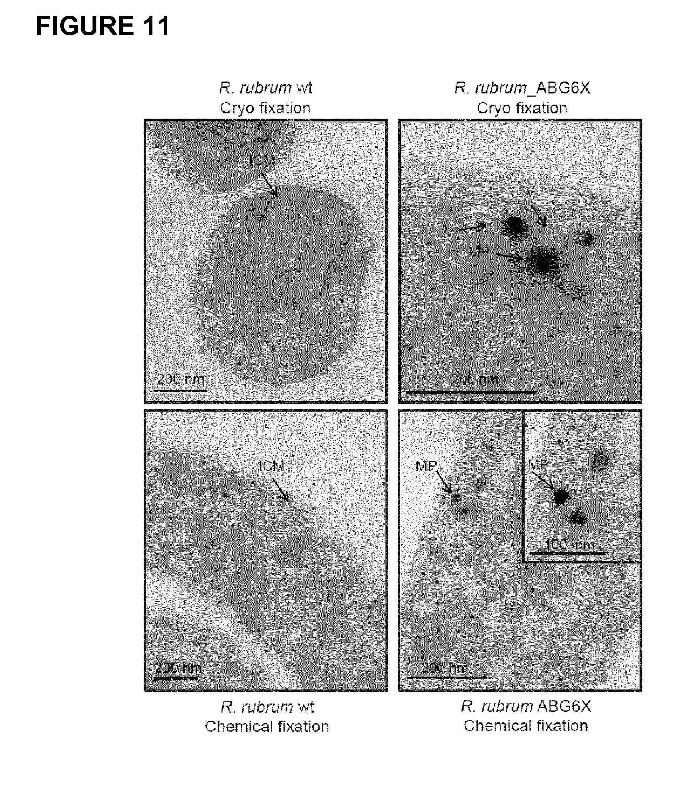

**FIGURE 11****: TEM of cryo-fixed or chemically fixed, thin sectioned *Rhodospirillum rubrum* strains.**
   Cells were cultivated under photoheterotrophic conditions prior to ultrastructural analysis. ICM sizes of cryo-fixed *R. rubrum* wt (93 ± 34 nm, n = 95) and vesicles surrounding immature magnetosomes of cryo-fixed *R*. *rubrum_ABG6X* (66 ± 6 nm, n = 6) were measured.

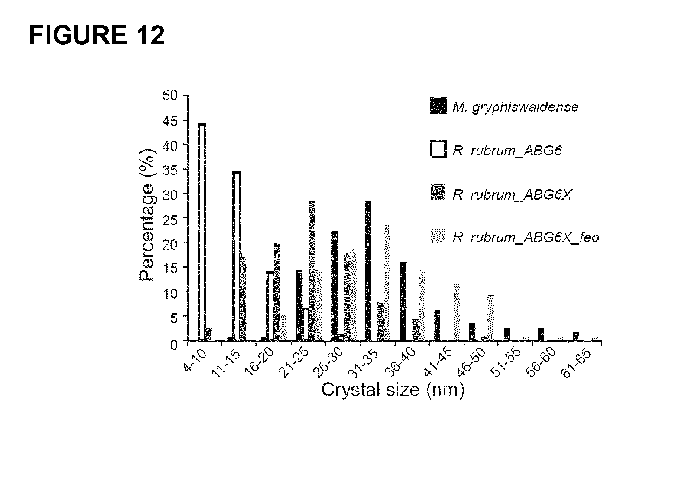

**FIGURE 12****: Size distribution of magnetosome crystals in *Magnetospirillum gryphiswaldense* and different *Rhodospirillum rubrum* strains.**
   Whereas crystals of *R. rubrum_ABG6* (n = 303) and *R. rubrum_ABG6X* (n = 306) were smaller than those of the donor *M*. *gryphiswaldense* (n = 310), crystal sizes of *R. rubrum_ABG6X_feo* (n = 301) were significantly larger, approaching those of the donor strain (see also Table 1).
**FIGURE 13****: Transmission electron micrographs of whole cells of different *Rhodospirillum rubrum* strains expressing magnetosome gene clusters. Scale bar: 1 µm, inset: 0.2 µm.**

### DETAILED DESCRIPTION OF THE INVENTION

The present invention is related to the unexpected finding that gene expression cassettes encoding all relevant genes being sufficient for the biomineralization of bacterial magnetic nanoparticles could be successfully transferred, in their entirety, to a heterologous (non-magnetotactic) host cell and, upon expression, resulted in the synthesis of functional magnetic nanoparticles.

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In one aspect, the present invention relates to a recombinant host cell, comprising in its genome:
(i) a gene expression cassette capable of being expressed in the host cell, the gene expression cassette encoding a nucleic acid sequence encompassing at least 80% of the full-length sequence of the *mamAB* operon of a magnetotactic alphaproteobacterium;
(ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium; and
(iii) a gene expression cassette capable of being expressed in the host cell, the gene expression cassette encoding a nucleic acid sequence encompassing at least 80% of the full-length sequence of the *mms6* operon of a magnetotactic alphaproteobacterium;
wherein the recombinant host cell, upon expression of the gene expression cassettes in their entirety, is capable of producing magnetic nanoparticles.

In preferred embodiments, the recombinant host cell as defined herein further comprises:
(iv) a gene expression cassette capable of being expressed in the host cell, the gene expression cassette encoding a nucleic acid sequence encompassing at least 80% of the full-length sequence of the *mamXY* operon of a magnetotactic alphaproteobacterium; and/or
(v) a gene expression cassette capable of being expressed in the host cell, the gene expression cassette encoding a nucleic acid sequence encompassing at least 80% of the full-length sequence of the *feoAB1* operon of a magnetotactic alphaproteobacterium.

The term "recombinant host cell", as used herein, refers to any type of host cells comprising in their genome (i.e. the lineom or the chromosome(s) as well as any additional episomal genetic entities such as vectors, plasmids, phagemids, phages, cosmids or artificial chromosomes) the above-referenced "*mamAB, mamGDFC, mms6* gene expression cassettes" and optionally also the *"mamXY* and/or *feoAB1* gene expression cassettes", these gene expression cassettes being introduced into the host cells by means of recombinant gene technology (see, e.g., Sambrook, J., and Russel, D.W. (2001) Molecular cloning: A laboratory manual (3rd ed.). Cold Spring Harbor, NY: Cold Spring Harbor Laboratory Press), such as transformation, transfection, conjugation or transduction. The term "recombinant host cell", as used herein, also refers to any host cells having endogenous copies of the *mamAB, mamGDFC, mms6, mamXY,* and *feoAB1* operon but further comprising (i.e. in addition to the endogenous copies) the recombinant (exogenous) gene expression cassettes as defined herein. The gene expression cassettes as defined herein may be maintained in the host cells as part of independent episomal genetic entities or stably integrated in the host cell's genome (i.e. the lineom or the chromosome(s)), with the latter alternative being preferred. Stable integration into the genome may be accomplished by homologous or non-homologous recombination or by transposition, all of them well established in the art (see, e.g., Sambrook, J., and Russel, D.W. (2001) *supra).*

In preferred embodiments, the recombinant host cells do not have endogenous copies of any one or more of the *mamAB, mamGDFC, mms6, mamXY,* and *feoAB1* operons (that is, any one, any two, any three, any four or all five). In other words, any one or more of gene expression cassettes as defined herein represent heterologous nucleic acid sequences with respect to the host cells employed.

In specific embodiments, the recombinant host cells to be employed are prokaryotic cells, such as bacterial or archeal cells, with bacterial cells being preferred. Examples of suitable prokaryotic cells include *inter alia E.* coli, and *Salmonella typhimurium.* In particular embodiments, the bacterial host cells are derived from an alphaproteobacterium, that is, any species belonging to one of the orders *Magnetococcales, Rhodobacterales, Rhodospirillales, Rickettsiales, Sphingomonadales, Caulobacterales, Kordiimondales, Parvularculales, Kiloniellales,* and *Sneathiellales* (Williams, K.P. et al. (2007) J. Bacteriol. 189, 4578-4586). In preferred embodiments, the bacterial host cells are derived from the order *Rhodospirillales,* more preferably from the genus *Rhodospirillum,* and particularly preferably from the species *Rhodospirillum rubrum (R. rubrum).*

In a particularly preferred embodiment, the recombinant host cell having stably integrated in its genome the expression cassettes (i) to (v) as described above relates to the host cell being deposited on November 14, 2013 under the Budapest Treaty at the *Deutsche Sammlung von Mikroorganismen und Zellkulturen* (Braunschweig, Germany) having the accession number DSM 28038. The deposited recombinant host cell has the taxonomic designation *Rhodospirillum rubrum* ATCC 11170 (which is herein also referred to as *Rhodospirillum rubrum_ABG6X_feo*).

In further preferred embodiments, the bacterial host cells are derived from the order *Magnetococcales* (herein also referred to as "magnetotactic alphaproteobacteria") more preferably from the genus *Magnetospirillum,* and particularly preferably from the species *Magnetospirillum gryphiswaldense* (*M*. *gryphiswaldense*). Further suitable host cells include *M*. *magneticum, M*. *magnetotacticum,* MV-1, and MC-1.

In specific embodiments, the recombinant host cells to be employed are eukaryotic cells, such as yeast (e.g., *Saccharomyes spec.), Pichia pastoris*, and mammalian cells, with the latter ones being preferred. Examples of mammalian cells include mouse, rat, hamster, rabbit, cat, dog, pig, cow, horse, monkey, and particularly preferably human cells.

The term "magnetic nanoparticles", as used herein, denotes any particles having a size in the nanometer scale that exhibits magnetic properties (i.e. that orients in a magnetic field along the magnetic field lines), either being ferromagnetic or being superparamagnetic or exhibiting an intermediate characteristic. The term "nanometer scale", as used herein, refers to a particle diameter of less than 1000 nm (i.e. 1 µm), preferably of less than 500 nm, and particular preferably of less than 100 nm. The "magnetic nanoparticles" may comprise one or more magnetic crystals but are preferably "mono-crystalline". In preferred embodiments, the magnetic nanoparticles further comprise an outer lipid bilayer membrane surrounding the one or more crystals (Grünberg, K. et al. (2004) Appl. Environ. Microbiol. 70, 1040-1050): Particles comprising a lipid bilayer membrane are also referred to as "magnetosomes".

The magnetic nanoparticles/magnetosomes being produced by the recombinant host cells employed herein may consist of of one or more metal oxides and/or metal sulfides, preferably of the elements in the forth row of the periodic table (i.e. chrome, manganese, iron, cobalt, and nickel). In particular embodiments, the magnetic nanocrystals are made of a single metal oxide or a single metal sulfide, preferably of an iron oxide such as hematite (Fe₂O₃) and magnetite (Fe₃O₄) or an iron sulfide such as greigite (Fe₃S₄). In particularly preferred embodiments, the magnetic nanoparticles produced according to the present invention consist of magnetite.

The term "capable of producing magnetic nanoparticles", as used herein, denotes the capacity of the recombinant host cells employed to synthesis, upon concomitant expression of the respective "*mamAB*, *mamGDFC, mms6* gene expression cassettes" and optionally also the *"mamXY* and/or *feoAB1* gene expression cassettes", functional magnetic nanoparticles as described above.

The term "gene expression cassette", as used herein, typically denotes a nucleic acid sequence comprising sequence elements which contain information regarding to transcriptional and/or translational regulation, such regulatory sequences being "operably linked" to the nucleotide sequence encoding one or more polypeptides to be expressed (i.e. to be synthesized starting from the gene sequence). An operable linkage is a linkage in which the regulatory sequence elements and the sequence to be expressed (and/or the sequences to be expressed among each other) are connected in a way that enables gene expression. The precise nature of the regulatory regions necessary for gene expression may vary among species, but in general these regions comprise a promoter which, in prokaryotes, contains both the promoter *per se,* i.e. DNA elements directing the initiation of transcription, as well as DNA elements which, when transcribed into RNA, will signal the initiation of translation. Such promoter regions normally include 5' non-coding sequences involved in initiation of transcription and translation, such as the -35/-10 boxes and the Shine-Dalgarno element in prokaryotes or the TATA box, CAAT sequences, and 5'-capping elements in eukaryotes. These regions can also include enhancer or repressor elements as well as translated signal and leader sequences for targeting the native polypeptide to a specific compartment of a host cell. In addition, the 3' non-coding sequences may contain regulatory elements involved in transcriptional termination, polyadenylation or the like. If, however, these termination sequences are not satisfactory functional in a particular host cell, then they may be substituted with signals functional in that cell.

The term "operon", as used herein, refers to a functional genetic unit of two or more genes, which are transcribed together into a single mRNA and are typically under the control of common regulatory sequence elements (i.e. promoters, terminators, and the like).

The *mamAB, mamGDFC, mamXY, mms6* and *feoAB1* operons encoded by the respective gene expression cassettes as described herein are derived from a "magnetotactic alphaproteobacterium" (cf. the definition herein above), more preferably from the genus *Magnetospirillum,* and particularly preferably from the species *M. gryphiswaldense.* The exact nucleotide sequences of all these operons are known in the art and can be accessed under GenBank accession number AM085146.1 (relating to the complete 130 kb sequence of the magnetosome island of *M. gryphiswaldense* MSR-1; cf. also Ullrich, S. et al. (2005) J. Bacteriol. 187, 7176-7184). In specific embodiments, the *mamAB, mamGDFC, mamXY, mms6* and *feoAB1* operons encoded by the respective gene expression cassettes present in a particular host cell are all derived from the same magnetotactic alphaproteobacterium, for example from *M. gryphiswaldense.* In other specific embodiments, the operons encoded by the respective gene expression cassettes present in a particular host cell are derived from two or more different magnetotactic alphaproteobacteria, for example *mamAB* and *mamGDFC* from *M. gryphiswaldense,* and the remaining operons from *M. magnetotacticum.*

Each of the gene expression cassettes (that is, independently from each other) introduced into the genome of the recombinant host cell encode nucleic acid sequences encompassing at least 80% of the full-length sequence of the respective operon sequences. Sequence identity is calculated over the entire length of the full-length sequence of the respective operon, that is, the sequence present in each of the gene expression cassettes may be terminally truncated (either 5'-terminally or 3'-terminally or 5'- and 3'-terminally) and/or may include one or more deletions or substitutions of internal sequences (e.g., the deletion and/or replacement of one or more genes comprised in a given operon).

In preferred embodiments, one or more of the gene expression cassettes encode nucleic acid sequences encompassing at least 85%, at least 90% or at least 95% of the full-length sequence of the respective operon sequences. In particular embodiments, the one or more of the gene expression cassettes encode nucleic acid sequences encompassing the full-length sequence of the respective operon sequences. In specific embodiments, one or more of the gene expression cassettes encode nucleic acid sequences encompassing at least 50%, at least 60% or at least 70% of the full-length sequence of the respective operon sequences. Sequence identity may be determined based on the nucleotide sequence or on the amino acid sequence. Various software tools and algorithms for determining nucleotide sequence and/or amino acid sequence identity are well established in the art (e.g., BLAST, FASTA, ClustalW, UGENE).

For example, in one embodiment, the present invention relates to a recombinant host cell, comprising in its genome: a gene expression cassette capable of being expressed in the host cell, the gene expression cassette encoding a nucleic acid sequence encompassing at least 80% of the full-length sequence of the *mamAB* operon of *M. gryphiswaldense*; a gene expression cassette capable of being expressed in the host cell, the gene expression cassette encoding a nucleic acid sequence encompassing at least 90% of the full-length sequence of the *mamGDFC* operon of *M. gryphiswaldense*; and a gene expression cassette capable of being expressed in the host cell, the gene expression cassette encoding a nucleic acid sequence encompassing the full-length sequence of the *mms6* operon of *M. gryphiswaldense.* In another, embodiment, the present invention relates to a recombinant host cell, comprising in its genome: a gene expression cassette capable of being expressed in the host cell, the gene expression cassette encoding a nucleic acid sequence encompassing at least 50% of the full-length sequence of the *mamAB* operon of *M. gryphiswaldense*; a gene expression cassette capable of being expressed in the host cell, the gene expression cassette encoding a nucleic acid sequence encompassing at least 80% of the full-length sequence of the *mamGDFC* operon of *M. gryphiswaldense*; and a gene expression cassette capable of being expressed in the host cell, the gene expression cassette encoding a nucleic acid sequence encompassing at least 90% of the full-length sequence of the *mms6* operon of *M. gryphiswaldense.*

In another embodiment, the present invention relates to a recombinant host cell, comprising in its genome:
(i) a gene expression cassette capable of being expressed in the host cell, the gene expression cassette encoding a nucleic acid sequence encompassing at least 80% of the full-length sequence of the *mamAB* operon of a magnetotactic alphaproteobacterium;
(ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium;
(iii) a gene expression cassette capable of being expressed in the host cell, the gene expression cassette encoding a nucleic acid sequence encompassing at least 80% of the full-length sequence of the *mms6* operon of a magnetotactic alphaproteobacterium; and
(iv) a gene expression cassette capable of being expressed in the host cell, the gene expression cassette encoding a nucleic acid sequence encompassing at least 80% of the full-length sequence of the *mamXY* operon of a magnetotactic alphaproteobacterium;
wherein the recombinant host cell, upon expression of the gene expression cassettes in their entirety, is capable of producing magnetic nanoparticles.

In another embodiment, the present invention relates to a recombinant host cell, comprising in its genome:
(i) a gene expression cassette capable of being expressed in the host cell, the gene expression cassette encoding a nucleic acid sequence encompassing at least 80% of the full-length sequence of the *mamAB* operon of a magnetotactic alphaproteobacterium;
(ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium;
(iii) a gene expression cassette capable of being expressed in the host cell, the gene expression cassette encoding a nucleic acid sequence encompassing at least 80% of the full-length sequence of the *mms6* operon of a magnetotactic alphaproteobacterium; and
(v) a gene expression cassette capable of being expressed in the host cell, the gene expression cassette encoding a nucleic acid sequence encompassing at least 80% of the full-length sequence of the *feoAB1* operon of a magnetotactic alphaproteobacterium;
wherein the recombinant host cell, upon expression of the gene expression cassettes in their entirety, is capable of producing magnetic nanoparticles.

In a preferred embodiment, the present invention relates to a recombinant host cell, comprising in its genome:
(i) a gene expression cassette capable of being expressed in the host cell, the gene expression cassette encoding a nucleic acid sequence encompassing at least 80% of the full-length sequence of the *mamAB* operon of a magnetotactic alphaproteobacterium;
(ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium;
(iii) a gene expression cassette capable of being expressed in the host cell, the gene expression cassette encoding a nucleic acid sequence encompassing at least 80% of the full-length sequence of the *mms6* operon of a magnetotactic alphaproteobacterium;
(iv) a gene expression cassette capable of being expressed in the host cell, the gene expression cassette encoding a nucleic acid sequence encompassing at least 80% of the full-length sequence of the *mamXY* operon of a magnetotactic alphaproteobacterium; and
(v) a gene expression cassette capable of being expressed in the host cell, the gene expression cassette encoding a nucleic acid sequence encompassing at least 80% of the full-length sequence of the *feoAB1* operon of a magnetotactic alphaproteobacterium;
wherein the recombinant host cell, upon expression of the gene expression cassettes in their entirety, is capable of producing magnetic nanoparticles. In a particularly preferred embodiment, the gene expression cassettes (i) to (v) all encode nucleic acid sequences encompassing the full-length sequences of the respective operons.

The gene expression cassettes (i) to (v) as described above may be provided independently or any two, any three, any four or all five gene expression cassettes may be provided in combination (e.g. as part of a common genetic vector or transposon). For example, the gene expression cassettes (i), (ii), and (iii) may be provided in combination.

Preferably, the combination of the gene expression cassettes (i), (ii), and (iii) has a nucleic acid sequence as shown in SEQ ID NO. 35. Also preferably, gene expression cassette (iv) has a nucleic acid sequence as shown in SEQ ID NO. 36 and/or gene expression cassette (v) has a nucleic acid sequence as shown in SEQ ID NO. 37.

The total length of the gene expression cassettes (i) to (iii), and optionally also (iv) and/or (v) comprised in the genome of the recombinant host cell (each being present in single copy) is less than 100 kb, preferably less than 80 kb or less than 60 kb, and particularly preferably less than 40 kb or less than 35 kb.

The term "gene expression cassette capable of being expressed in the host cell", as used herein, denotes that the operon encoded by the gene expression cassette is under the control of regulatory elements being functional in the host cell employed. In preferred embodiments, gene expression of the respective operons is under the control of the endogenous regulatory elements, that is, the homologous regulatory elements naturally occurring in the particular organism from which the respective operon(s) is/are derived. For example, if the respective operons are derived from *M. gryphiswaldense,* the corresponding regulatory elements derived from the same organism may be employed. Typically, the gene expression cassettes may then comprise a nucleic acid sequence encompassing 100-800, preferably 200-600 nucleotides upstream (i.e. 5') of the respective operon and 100-800, preferably 200-600 nucleotides downstream (i.e. 3') of the respective operon. In other embodiments, the regulatory elements comprise non-endogenous sequences, that is exogenous inducible or constitutive promoters (e.g., tet, lac, CMV promoters) and terminators.

In further specific embodiments, the recombinant host cell comprises two or more copies of any one or more of the gene expression cassettes. Preferably, the copy numbers of all gene expression cassettes being present in a recombinant host cell are identical, for example two copies. In other embodiments, the copy numbers of the respective gene expression cassettes are different.

In a further aspect, the present invention relates to a method for the production of a recombinant host cell as defined herein, the method comprising the transfer of the gene expression cassettes into the host cell by means of genetic transposition, and in particular comprising a modular transfer of the gene expression cassettes.

The gene expression cassettes (i) to (v) as described above may be introduced in to the recombinant host cells independently or any two, any three, any four or all five gene expression cassettes may be introduced concomitantly on a common genetic vector or transposon.

In yet another aspect, the present invention relates to the use of a recombinant host cell as defined herein for the biotechnological production of magnetic nanoparticles.

In yet another aspect, the present invention relates to the use of a recombinant host cell as defined herein for the *in vivo* synthesis of magnetic nanoparticles for application in magnetogenetics or biomedical imaging.

The invention is further described by the figures and the following examples, which are solely for the purpose of illustrating specific embodiments of this invention, and are not to be construed as limiting the claimed subject matter in any way.

### EXAMPLES

### 1. Materials and methods

### 1.1 Bacterial strains

Bacterial strains are shown in **Table 4.** *E. coli* strains were cultivated as previously described (Sambrook, J. & Russell, D. (2001) Molecular cloning: a laboratory manual. Vol. 3, Cold Spring Harbor Laboratory Press). 1 mM DL-α,ε-diaminopimelic was added for growth of BW29427 and WM3064.

Liquid cultures and single colonies of *Magnetospirillum gryphiswaldense* were cultivated in FSM medium or on agar plates (1.5% agar) incubated at 30°C under microaerobic (1% O₂) conditions (Heyen, U. and Schüler, D. (2003) Appl. Microbiol. Biotechnol. 61, 536-544; Kolinko, I. et al. (2011) J. Bacteriol. 193, 5328-5334)

Cultures of *Rhodospirillum rubrum* strains were cultivated chemoheterotrophically (ATCC medium 112) with free gas exchange in the dark, microaerobically in M2SF medium (Ghosh, R. et al. (1994) Appl. Environ. Microbiol. 60, 1698-1700), or photoheterotrophically in Sistrom A medium (Sistrom, W. R. (1962) J. Gen. Microbiol. 28, 607-616) supplemented with Fe(III) citrate or Fe(II) sulfate at 18°C, 23°C or 30°C (10, 400, 1000, 9000 lux). The 16S rRNA similarity to M. gryphiswaldense is shown in **Table 2.**

### 1.2 Plasmid construction and conjugative transfer

Plasmids and oligonucleotides used are shown in **Table 4** and **Table 5,** respectively. Red/ET recombination was performed as previously described (Wang, J. et al. (2006) Mol. Biotechnol. 32, 43-53 (2006); Fu, J. et al. (2008) Nucl. Acids Re.s 36, e113) in order to construct expression cassettes harboring a mariner transposon vector backbone and magnetosome operons obtained from a BAC sequence pSSK18 (Schübbe, S. et al. (2003) J. Bacteriol. 185, 5779-5790) (*mamAB*) and *M. gryphiswaldense* genomic DNA *(mamGFDC, mms6, mamXY*). Briefly, a cloning cassette was amplified by polymerase chain reaction (PCR) and transferred into electro-competent *E. coli cells* (DH10b) expressing phage-derived recombinases from a circular plasmid (pSC101-PBad-gbaA). After transfer of the cassette, recombination between homologous regions on the linear fragment and the plasmid occurred. For introducing of the magnetosome gene operons (cf. **FIG. 4****),** triple recombination was used with cotransforming of two linear fragments, which recombined with a circular plasmid (Fu; j. et al. (2008) Nucleic Acids Res. 36, e113). Recombinants harboring the correct plasmids were selected by restriction analysis.

For construction of plasmids harboring fusion proteins, the *mamDC* promoter *(Xbal, BamHI* restriction sites added), *mamGFDC* operon or *mamJ* gene *(Ndel, KpnI*) and *egfp* (*KpnI, EcoRI*) were cloned into a pBAM1 plasmid (Martinez-Garcia, E. et al. (2011) BMC Microbiol. 11, 38) with a tetracycline resistance cassette (exchange of *km^{R}* against *tc^{R}* with *SanDI* and *AatlI*). The replicative plasmid pFM211 (obtained from Dr. Frank Müller, Ludwig-Maximilians-Universität München, Germany, unpublished) harboring *ftsZm* with a *mCherry* fusion under control of an inducible *lac* promoter was recombined with pBAM1 to construct pBAM-ftsZm_mcherry. The resident *km^{R}* exchanged by *tc^{R}* using ET-recombination. For construction of pBAM_feoAB1, a fragment with P_{mamH} and *feoAB1* was amplified with PCR from pRU1feoAB (*XbaI, EcoRI*) and cloned into Tet-pBAM1. Conjugation experiments of *M. gryphiswaldense* deletion mutants (Lohsse, A. et al. (2011) PLoS One 6, e25561) were performed as described (Kolinko, I. *et al.* (2011) *supra).*

For conjugation of *R. rubrum,* cultures were incubated in ATCC 112 medium. Approximately 2*10⁹ cells were mixed with 1*10⁹ *E. coli cells,* spotted on ATCC agar medium and incubated for 15h. Cells were flushed from the plates and incubated on ATCC 112 agar medium supplemented with appropriate antibiotics for 7-10 days (Tc (tetracycline): 10 µg/ml, Km (kanamycin: 20 µg/ml, Gm (gentamicin): 10 µg/ml). Sequential transfer of the plasmids resulted in 10⁻⁶-10⁻⁸ antibiotic-resistant transconjugants per recipient, respectively. Two clones from each conjugation experiments were chosen for further analyses.

Transposition of the cassettes resulted in single-copy integration into the genome and stable maintenance for at least 40 generations in the absence of selection characterized transconjugants were indistinguishable from WT with respect to motility, cell morphology or growth during photoheterotrophic cultivation **(****FIG. 8****).**

### 1.3 Analytical methods

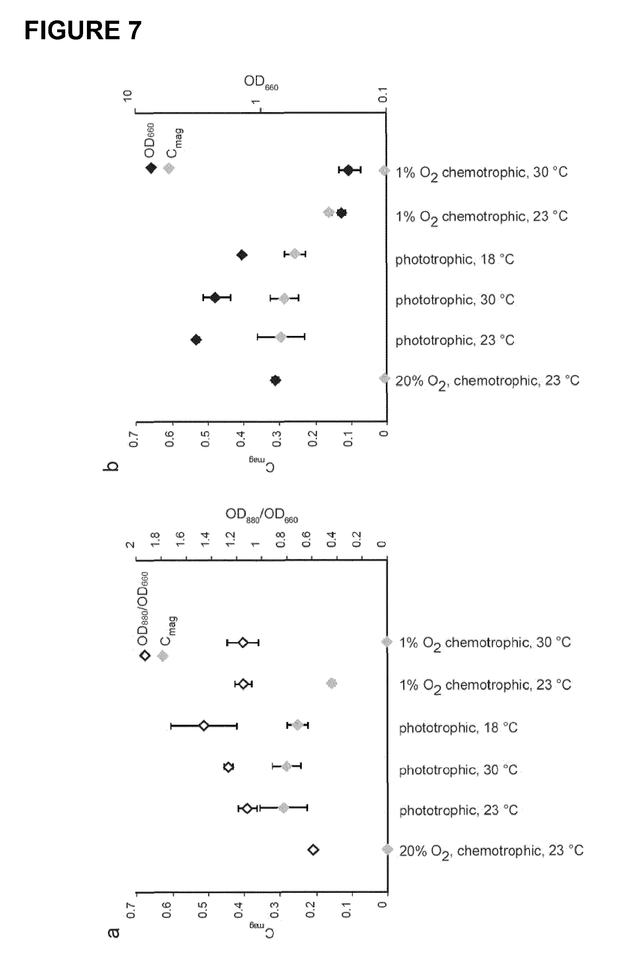

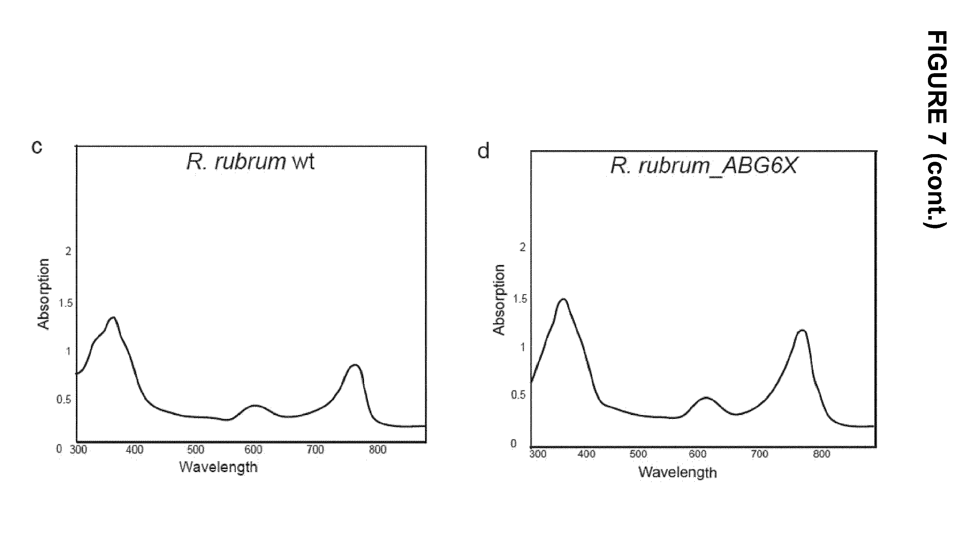

Optical density of *M. gryphiswaldense* cultures was measured at 565 nm (Schüler, D. et al. (1995) FEMS Microbiol. Ecol. 132, 139-145). Optical density of *R. rubrum* cultures was measured at 660 nm (minimal absorption of bacteriochlorophyll a (Bchl a)) and 880 nm (maximal Bchl a absorption) (Ghosh, R. *et al.* (1994) *supra).* The ratio of 880/660 nm was used to determine yields of chromatophores within intact cells. Furthermore, Bchl a was extracted from cultures with methanol and absorption spectra were measured in an Ultrospec 3000 photometer (GE Healthcare). The spectra of photoheterotrophically cultivated R. *rubrum_ABG6X* cells were indistinguishable from those of the wild-type **(****FIG. 7****).**

Intracellular iron concentrations were determined after incubation under anaerobic conditions using a modified version of the ferrozine assay (Viollier, E. et al. (2000) Appl. Geochemistry 15, 785-790). In brief, 4 ml cultures were centrifuged for 1 min at 11.000 rpm and resuspended in 90 µl HNO₃ (65%) for 3 h at 99 C. The average magnetic orientation of cell suspensions (magnetic response) was assayed with a light scattering assay as previously described (Schüler, D. *et al.* (1995) *supra).* Briefly, cells were aligned at different angles to a light beam by application of an external magnetic field. Crystal size (n = 120) and vesicle measurements (n = 100) were performed with ImageJ software.

### 1.4 Sequencing

Genomic sequencing of strain *R. rubrum_ABG6X* was performed via Next-Generation Sequencing by using the SOLID EZ Bead System (Life Technologies GmbH, Darmstadt, Germany) according to the manufacturer's protocol. No mutations were detected, except for a deletion (aa 169-247) within the hypervariable non-essential CAR domain of *mamJ.*

### 1.5 Microscopy

For transmission electron microscopy (TEM), cells and isolated magnetosomes were absorbed on carbon-coated copper grids. Purified magnetosomes were stained with 1% phosphotungstic acid (PTA) or 2% Uranyl acetate (UrAc). Samples were viewed and recorded with a Morgagni 268 microscope. Crystal sizes and Vesicle sizes were determined using ImageJ software.

Chemical fixation, high pressure freezing and thin sectioning of cells was performed as previously described (Jogler, C. et al. (2011) Proc. Natl. Acad. Sci. USA 108, 1134-1139). In brief, for chemical fixation, cells were incubated in 2.5% glutardialdehyde containing fixative buffer (75 mM sodium cacodylate, 2 mM MgCl₂, pH 7.0), for 1 h at room temperature. Afterward, samples were rinsed several times in fixative buffer and post-fixed at room temperature for 1 h with 1% osmium tetroxide in fixative buffer. After two washing steps in water, the cells were stained enbloc for 30 min with 1% uranyl acetate in 20% acetone. Dehydration was performed with a graded acetone series. Samples were then in-filtrated and embedded in Spurr's low-viscosity resin. For high-pressure freezing, aluminum platlets were filled with concentrated cell suspensions and the cells immobilized by high-pressure freezing (Leica; HPM100). Freeze substitution was performed in acetone with 2% osmium tetroxide and 0.2% uranyl acetate, including 5% water. After embedding the samples in Epon, ultrathin sections were cut with a diamond knife and mounted onto uncoated copper grids. The sections were post-stained with aqueous lead citrate (100 mM, pH 13.0. Micrographs were taken with an EM 912 electron microscope (Zeiss) equipped with an integrated OMEGA energy filter operated at 80 kV in the zero loss mode). Vesicle sizes were determined using ImageJ software.

High-resolution TEM (HRTEM) was performed using a JEOL 3010 microscope, operated at 297 kV and equipped with a Gatan Imaging Filter (GIF) for the acquisition of electron energy-loss spectra and energy-filtered compositional maps. For TEM data processing and interpretation the DigitalMicrograph and SingleCrystal software were used (Uebe, R. et al. (2011) Mol. Microbiol. 82, 818-835).

Cryo-electron-tomography was performed as previously described (Katzmann, E. et al. (2011) Mol. Microbiol. 82, 1316-1329). In brief, a FEI Tecnai F30 Polara transmission electron microscope was used. All data collection was performed at 300 kV, with the energy filter operated in the zero-loss mode (slit width of 20 eV). Tilt series were acquired using Serial EM and FEI software. Quantifoil copper grids (Quantifoil Micro Tools GmbH, Jena) were prepared by placing a droplet of 10-15 nm colloidal gold clusters (Sigma) on each grid for subsequent alignment purposes. Additionally a droplet of culture was added onto the prepared grid, and after blotting embedded in vitreous ice by plunge freezing into liquid ethane (temperature -170°C). The specimen was tilted typically about one axis with 1.5° increments over a total angular range of +/-65° to minimize the electron dose. The total dose accumulated during the tilt series was kept below 200 e/Å². To account for the increased specimen thickness at high tilt angles, the exposure time was multiplied by a factor of 1/cos α. The pixel size in unbinned images was 0.805 at 27500 magnification and 1.10 at 41.000 magnification.

Fluorescence microscopy was performed with an Olympus BX81 microscope equipped with a Hamamatsu Orca-ER camera. Exposure time of 0.12-0.25 s was used. Image scaling and cropping was performed with Photoshop 9.0 software.

### 1.6 Isolation of magnetic particles, electrophoresis and immunochemical detection

Unless described otherwise, expression of magnetic particles in *R. rubrum* was performed under photoheterotrophic conditions at 23°C and a light intensity of 1000 lux. Cells were harvested, washed and resuspended. Cell suspensions were lysed by sonification and cellular debris was removed by low-speed centrifugation.

Crystals were isolated from sonification-disrupted *R. rubrum_ABG6X_feo* cells using magnetic separation (Uebe, R. et al. (2011) Mol. Microbiol. 82, 818-835). For TEM analysis, whole cells were directly absorbed on carbon-coated copper grids. Alternatively, cells were chemically fixed or high pressure frozen and thin sectioned prior to ultrastructural analysis (Jogler, C. et al. (2009) Environ. Microbiol. 11, 1267-1277). Electron dense particles of *R. rubrum* WT, *R. rubrum_ABG, R. rubrum_ABG6* and *R. rubrum_ABG6X* were analyzed by HRTEM (Uebe, R. et al. (2011) Mol. Microbiol. 82, 818-835). Cryo-electron tomography was performed with cultures of *R. rubrum_ABG6X* or isolated magnetic particles from *R. rubrum_ABG6X_feo* as previously described (Katzmann, E. *et al.* (2011) *supra).* Magnetic particles were stained with 1% phosphotungstic acid (PTA) or 2% uranyl acetate (UrAc) to visualize organic material surrounding magnetic particles. Proteins of the organic layer were solubilized as previously described (Grünberg, K. et al. (2004) Appl. Environ. Microbiol. 70, 1040-1050; Lang, C. and Schüler, D. (2008) Appl. Environ. Microbiol. 74, 4944-4953) and analyzed by Western blotting and LC tandem MS.

Polyacrylamide gels were prepared according to the procedure of Laemmli (Laemmli, U. K. (1970) Nature 227, 680-685). Protein samples from different cellular fractions were resuspended in electrophoresis sample buffer and denatured at 98°C for 5 min (Uebe, R. *et al.* (2011) *supra).* 10 µg of protein extracts were separated on a 15% SDS-polyacrylamide gel. Protein bands were visualized by Coomassie brilliant blue staining. Western blot analysis for detection of MamC was performed as previously described (Lang, C. and Schüler, D. (2008) *supra).*

### 1.7 Mass spectrometric analysis

For MS analyses, 25 µg solubilized proteins were tryptically in-gel digested as described previously (Klein, A. et al. (2012) J. Cell. Biol. 199, 599-611). For protein identification and quantification, the tryptic fragments were separated on a C18 reversed-phase column with a linear acetonitrile gradient and analyzed by nano-electrospray ionization-LC tandem MS (ESI-LC-MS/MS), recorded on an Orbitrap mass spectrometer. Resulting spectra were analyzed via Mascot™ software using the National Center for Biotechnology Information (NCBI) nr Protein Database and a database from *M. gryphiswaldense* (Richter, M. et al. (2007) J. Bacteriol. 189, 4899-4910).

### 2. Heterologous production of magnetosomes in Rhodospirillum rubrum

In the alphaproteobacterium *Magnetospirillum gryphiswaldense* and related magnetotactic bacteria, biogenesis of functional magnetosomes is highly complex and involves the invagination of magnetosome vesicles from the cytoplasmic membrane, the magnetosomal uptake of iron and the crystallization of magnetite particles, as well as their assembly into chains along a dedicated cytoskeletal structure (Komeili, A. et al. (2006) Science 311, 242-245; Katzmann, E. et al. (2010) Mol. Microbiol. 77, 208-224). Recently, genes controlling magnetosome synthesis within several clusters of a larger (115 kb) genomic magnetosome island (MAI) were discovered, which are interspersed by transposases and genes of unknown function (Jogler, C. *et al.* (2009) *supra;* Jogler, C. *et al.* (2011), *supra;* Lefevre, C.T. et al. (2013) Environ. Microbiol., April 1, doi: 10.1111/1462-2920.12128). Whereas the smaller *mamGFDC* (2.1 kb), *mms6* (3.6 kb) and *mamXY* operons (5.1 kb) have accessory functions in biomineralization of properly sized and shaped crystals, the large *mamAB* operon (16.4 kb) encodes proteins essential for iron transport, magnetosome membrane assembly/formation, and crystallization of magnetosome particles as well as their assembly and intracellular positioning.

Using recombinant engineering based on phage driven homologous recombination (Zhang, Y. et al. (2000); Nat Biotechnol 18, 1314-1317; Fu, J. et al. (2008) Nucl. Acids Res. 36, e113), several compact modular expression cassettes were put together comprising all 29 genes (about 26 kb in total) of the four major/relevant operons in various combinations, but lacking the tubulin-like *ftsZm.* This gene was omitted from its native *mamXY* operon because of its observed interference with cell division during cloning. Regions 200-400 bp upstream of all operons were retained to ensure transcription from their native promoters (Schübbe, S. et al. (2006) Appl Environ Microbiol 72, 5757-5765) **(****FIG. 4****).**

Since in initial attempts conjugational transfer of multicopy plasmids harboring large parts of the mamAB operon resulted in lack of functional expression and genetic instability due to extensive rearrangements and deletions (data now shown), transposition cassettes comprising the MycoMar transposase gene *(tps)* or Tn5 transposase gene, two corresponding inverted repeats, the origin of transfer *oriT,* and an antibiotic resistance gene were designed to enable transfer and chromosomal integration of the gene clusters in single copy. Chromosomal re-integration of all cassettes into different non-magnetic, single-gene as well as large operon deletion strains of *M. gryphiswaldense* resulted in stable and complete restoration of magnetosome biomineralization **(****FIG. 5****).**

Chromosomal re-integration of all cassettes into different non-magnetic single-gene and operon deletion strains of *M. gryphiswaldense* resulted in stable wild-type like restoration of magnetosome biomineralization, indicating that transferred operons maintained functionality upon cloning and transfer **(****FIG. 5****).**

Next, the transfer of expression cassettes to a foreign non-magnetic organism was analyzed. The photosynthetic alphaproteobacterium *Rhodospirillum rubrum* was chosen as potential expression host due to the phylogenetic relationship between the *Rhodospirillaceae* and magnetotactic bacteria, which except magnetosome and photosynthetic gene functions, shares a high proportion of conserved genes (Richter, M. et al. (2007) J Bacteriol 189, 4899-4910) **(****FIG. 1**).

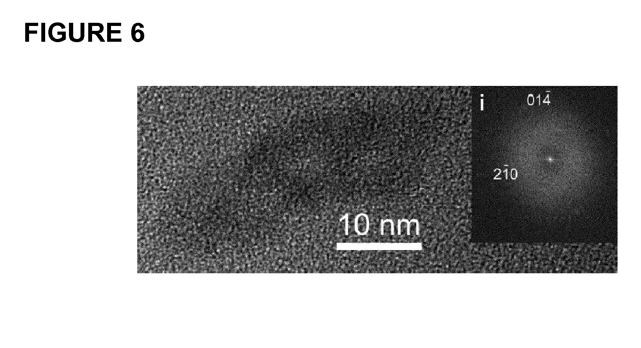

Neither conjugational transfer of the *mamAB* operon alone (pTps_AB), which was reported to be sufficient for weak magnetosome biomineralization in its native host, nor in combination with the accessory *mamGFDC* genes (pTps_ABG) had any detectable phenotypic effect on cells with respect to iron content and magnetic response C_{mag} (cf. **TABLE 1**). However, although no magnetic response after insertion of pTps_ABG6 (*mamAB*+*mamGFDC*+*mms6* operons) could be detected, the cellular iron content of *R. rubrum_ABG6* was increased 2.4-fold compared to the untransformed (i.e., wild-type) *R. rubrum.* Transmission electron micrography revealed the presence of irregularly shaped, flake-like electron dense particles (-12 nm) traversing the cell in a chain-like structure **(****FIG. 2a ii****),** which were identified as poorly crystalline hematite (Fe₂O₃) by analysis of lattice spacings in high-resolution TEM images (cf. **FIG. 6****),** much as in the hematite particles previously identified in M. *gryphiswaldense* mutants affected in crystal formation (Uebe, R. et al. (2011) Mol. Microbiol. 82, 818-835)

Next, in order to further enhance biomineralization pTps_XY was transferred into *R. rubrum_ABG6,* resulting in strain *R. rubrum_ABG6X* harboring, in addition to *mamAB, mamGFDC,* and *mms6,* also *mamXYZ,* thus encompassing all 29 relevant MAI (magnetosome island) genes, except for fr*ftsZm*. Intriguingly, cells of this strain exhibited a magnetic response in the classical light scattering assay for *Magnetospirillum* (C_{mag} ≈ 0.3) (Schüler, D. *et al.* (1995) *supra)* and were "magnetotactic" **(TABLE 1**), i.e., within several hours accumulated as a visible pellet near the pole of a permanent magnet at the edge of a culture flask **(****FIG. 2b****).** TEM revealed the presence of cuboidal electron dense particles with an average size of 25 nm, which were aligned in short, fragmented chains loosely dispersed within the cells **(****FIG. 2a iii****).** Electron diffraction identified the electron dense particles as single or twinned crystals of magnetite (Fe₃O₄) **(****FIG. 11**). Despite their smaller sizes (average: 24 nm) the particles strongly resembled the magnetosomes of the donor strain with respect to their projected outlines and thickness contrast, suggestive of cubooctahedral or octahedral crystal morphologies **(****FIG. 2d****).**

Separate insertion of the *ftsZm* gene, which was first omitted from pTps_XY because of its observed interference with cell division during cloning, under control of the inducible lac promoter did not further increase the intracellular iron content and number and size of the crystals compared to strains *R. rubrum_ABG6X* **(****FIG. 1a iv****; TABLE 1),** despite of an increased C_{mag}, possibly due to subtle effects on cell morphology. Although it was implicated in biomineralization in *M. gryphiswaldense* (Ding, Y. et al. (2010) J. Bacteriol. 192, 1097-1105), the tubulin-like FtsZm does not appear to have a crucial role for biomineralization in *R. rubrum.*

Subsequently, the relationship between magnetite formation and growth conditions of the metabolically versatile *R. rubrum* was investigated. During both photoheterotrophic and microoxic chemotrophic growth in the dark a magnetic response of strain *R. rubrum_ABG6X* was detectable. No magnetic response was detectable under aerobic conditions (**FIGs. 7** **and** **8****).** In contrast to extracellular iron speciation (ferric vs. ferrous iron at various concentrations), which did not affect particles formation, biomineralization was completely inhibited by higher temperatures (>30°C) under microoxic conditions, which was also observed after serial passages under phototrophic conditions. Phototrophic growth with moderate light intensity (1000 lux) at 23°C was found to support highest magnetic responses and reasonable/robust growth compared to low light conditions or high light intensity (<400 lux, 9000 lux). Therefore, cells were grown under these conditions in all subsequent experiments.

To test whether known mutation phenotypes from M. *gryphiswaldense* could be replicated in *R rubrum,* variants of expression cassettes were constructed, in which single genes were omitted from the *mamAB* operon by deletion within the cloning host E. coli. The small (77 amino acids) Maml protein was previously implicated in MM vesicle formation and found to be essential for magnetosome synthesis (Murat, D. et al. (2010) Proc. Natl. Acad. Sci. USA 107, 5593-5598). *R. rubrum_ABG6X-dl* failed to express magnetosome particles **(****FIG. 13****),** which resembles the phenotype of a *maml* deletion in the related *M. magneticum.* Another tested example was MamJ, which is assumed to connect magnetosome particles to the cytoskeletal magnetosome filament formed by the actin-like MamK (Scheffel, A. et al. (2006) Nature 440, 110-114). As in *M. gryphiswaldense,* deletion of *mamJ* caused agglomeration of magnetosome crystals in about 65% of *R. rubrum_ABG6X-dJ* cells (**FIGs. 2a v** **and** **13****, TABLE 1**). Altogether, these observations indicate that magnetosome biogenesis and assembly within the foreign host are governed by very similar mechanisms and structures as in the donor, which are conferred by the transferred genes.

In *M*. *gryphiswaldense* and other magnetotactic bacteria, magnetite synthesis occurs within intracellular vesicles surrounded by the magnetosome membrane (MM). In cells of *R. rubrum_ABG6X* vesicular structures surrounding small, immature magnetite crystals were occasionally detected in thin-sectioned cells or by cryo-electron tomography (CET) that, however, were diffcult to be distinguished from the highly abundant intracytoplasmic membranes (ICMs) having diameters of 93 ± 34 nm **(****FIG. 11****).** Particles that were isolated from disrupted cells by magnetic separation and centrifugation were clearly surrounded by a layer of organic material (3.6 ± 1.2 nm), resembling the magnetosome membrane attached to isolated magnetosomes of *M*. *gryphiswaldense* (3.2 ± 1.0 nm). This organic layer could also be seen by means of CET (3.4 ± 0.9 nm). Smaller, apparently immature crystals (19 ± 6 nm) were sometimes surrounded by partially empty vesicles (66 ± 6 nm) visible both in micrographs of negatively stained samples and cryo-electron tomograms **(****FIG. 11**). Although these vesicular structures overlapped with the reported size range of *R. rubrum* chromatophores (50-150 nm) (Cohen-Bazire, G. and Kunisawa, R. (1963) J Cell Biol 16, 401-419), no esicles larger than 100 nm surrounding the magnetic particles were detected, whereas 28% of ICM vesicles had a size of 100-170 nm. Biomineralization was severely impaired in the absence of ICM, as transfer of the entire gene set into an ICM deficient mutant of *R. rubrum* (strain GN21) resulted in a very weak magnetic response (C_{mag} = 0.02), with only a minor portion of cells (~10 %) harboring magnetic particles of 27 nm, if grown under microoxic conditions in the dark. Occasionally empty vesicles were observed in GN21_*ABG6X* (**FIG. 11**)**,** which were absent from untransformed cells. These observations seems to point towards a scenario in which the ability to synthesize magnetosome membrane-like structures is conferred by the transformed magnetosome operons, rather than adopting pre-existing chromatophore vesicles synthesis of magnetic particles.

Organic material potentially identical with a MM could be solubilized from isolated magnetite crystals of *R*. *rubrum_ABG6X* by 1% SDS, and less effective also by Triton X-100 treatment (**FIGs. 3** **and** **9**)**,** similar as reported for MM of *M*. *gryphiswaldense* (Grünberg, K. *et al.* (2004) *supra).* 1D SDS-PAGE of proteins that co-purified with magnetic particles resolved a complex pattern that displayed several bands of identical size also present in MM preparations from *M*. *gryphiswaldense* (**FIG.** 9, arrows). An antibody against MamC, the most abundant protein in the MM of *M*. *gryphiswaldense* (Grünberg, K. *et al.* (2004) *supra),* revealed a band of 12.4 kDa, which was absent from the soluble fraction, but also yielded a faint signal in the non-magnetic membrane fraction, possibly originating from incomplete magnetic separation of the particles during the isolation process or colocalization in empty membrane vesicles (**FIG. 9**).

In addition to several proteins characteristic for the specialized ICM systems of *R*. *rubrum* (LH complex, reaction center, cytochrome C, ATP synthases) (Smith, L. (1954) Bacteriol. Rev. 18, 106-130; Pace, G. W. et al. (1979) European J. Appl. Microbiol. Biotechnol. 6, 271-278 (1979); Wang, Z. Y. et al. (2005). J. Mol. Biol 347, 465-477), mass spectrometry identified several magnetosome proteins from *M. gryphiswaldense* (MamJ, MamK, MamC, MamD, MamE, MamO, MamH, MamY, Mms6, MamA, MmsF, MamF, MamM) among most abundant proteins in the putative MM). These findings might indicate a physical connection between magnetosomes and ICMs, or alternatively presence of highly abundant chromatophore proteins might simply result from cross-contaminantion during cell disruption. The subcellular localization of selected single magnetosome proteins in *R*. *rubrum* dependent on the presence of further determinants encoded by the transferred genes. Whereas GFP fusions to either MamJ, or MamC in displayed a dispersed pattern in the untransformed *R. rubrum,* a filamentous/chain-like fluorescent signal became apparent in the *R. rubrum_ABG6X* background in which the full complement of magnetosome genes is present, reminiscent to the magnetosome-chain localization of these proteins in *M*. gryphiswaldensis (Scheffel, A. et al. (2006) Nature 440, 110-114; Lang, C. and Schüler, D. (2008) *supra)* (**FIG. 8**).

In order to further verify whether phenotypic effects known from magnetosome gene mutations in *M. gryphiswaldense* could be replicated in *R. rubrum,* variants of the expression cassettes were constructed, in which single genes were deleted from the *mamAB* operon.

For example, *R. rubrum_ABG6X-dl* failed to express magnetic particles as revealed by C_{mag} and TEM, which phenotypicxally paralleled the deletion of *maml* in *M*. *magneticum* (Murat, D. et al. (2010) Proc. Natl. Acad. Sci. USA 107, 5593-5598). Another example is MamJ, which is assumed to connect magnetosome particles to the actin-like cytoskeletal magnetosome filament formed by MamK. Deletion of *mamJ* did not affect biomineralization, but caused agglomeration of magnetic particles in *R. rubrum_ABG6X-dJ* (**FIG. 3**), much as observed in *M. gryphiswaldense* (Scheffel, A. *et al.* (2006) *supra),* indicating that also the chain-like assembly of magnetosome crystals is genetically controlled in *R. rubrum_ABG6X* (**FIG. 13**).

The size of the magnetic particles in *R. rubrum_ABG6X* was still smaller compared to those formed in the donor organism *M*. *gryphiswaldense.* This raised the question whether full expression of biomineralization may depend on the presence of auxiliary gene functions, which were recently identified also outside the transferred gene clusters (refs nap, feo). Thus, we genetically introduced a ferrous iron transporter (FeoAB1), which has been found to exhibit accessory functions for magnetosome biomineralization (Rong, C. et al. (2008) Res. Microbiol. 159, 530-536). Genomic insertion of *feoAB1,* in addition to the *mamAB, mamGFDC, mms6* ,and *mamXY* operons, into strain *R. rubrum_ABG6X* resulted in strain *R. rubrum_ABG6X_feo,* which displayed a further increased iron content size of the particles increased significantly, approaching the crystal size of *M*. *gryphiswaldense.* (**FIG. 3**).

As magnetosomes in *R. rubrum_ABG6X were* still smaller than those of *M*. *gryphiswaldense,* it was asked whether full expression of biomineralization may depend on the presence of further auxiliary functions possibly encoded outside the canonical magnetosome operons. For instance, deletion of *feoB1* encoding a constituent of a MTB-specific ferrous iron transport system caused fewer and smaller magnetosomes in *M. gryphiswaldense* (Rong et al. (2008) *supra).* Strikingly, insertion of *feoAB1* into *R. rubrum* strain *ABG6X* resulted in even larger, single-crystalline and twinned magnetosomes and longer chains (440 nm) (**FIG**. **2a vi****, TABLE 1**). The size (37 nm) of the crystals approached that of the donor and cellular iron content was substantially increased (0.28% of dry weight) compared to *R. rubrum_ABG6X* (0.18%), although still lower than in *M. gryphiswaldense* (3.5%), partly owing to the considerably larger volume of *R. rubrum* cells (**FIG. 2c**).

Magnetosome particles could be purified from disrupted cells by magnetic separation and centrifugation and formed stable suspensions (**FIG. 3**). Isolated crystals were clearly enclosed by a layer of organic material resembling the MM attached to magnetosomes of *M. gryphiswaldense.* Smaller, immature crystals were surrounded by partially empty vesicles **(****FIG. 3c** **inset)** that were also seen in thin-sectioned cells **(****FIGs.** 3a and **11****)** and on average were smaller (66 ± 6 nm) than the abundant photosynthetic intracytoplasmic membranes (ICM) (93 ± 34 nm (**FIG. 11**). Organic material of the putative MM could be solubilized from isolated magnetite crystals of *R. rubrum_ABG6X* by various detergents (**FIG. 3d**), similar as reported for MM of M. *gryphiswaldense* (Grünberg et al. (2004) *supra).* Proteomic analysis of the SDS-solubilized MM revealed a complex composition (**FIG. 9**), and several genuine magnetosome proteins (MamKCJAFDMBYOE, Mms6, MmsF) were detected among the most abundant polypeptides (**TABLE 3**). An antibody against MamC, the most abundant protein in the MM of *M*. *gryphiswaldense,* recognized a prominent band with the expected mass (12.4 kDa) also in the MM of *R. rubrum_ABG6X* (**FIG. 9**).

The subcellular localization of selected magnetosome proteins in *R. rubrum* depended on the presence of further determinants encoded by the transferred genes. For example, GFP fusions of MamC displayed a punctuate pattern in the *R. rubrum* wild-type background. In contrast, a filamentous fluorescent signal became apparent in the majority of cells (79%) of the *R. rubrum_ABG6X* background in which the full complement of magnetosome genes is present (FIG. 10), reminiscent of the magnetosome-chain localization of these proteins in *M*. *gryphiswaldense.*

The heterologous production of magnetic nanoparticles in *R. rubrum* should result in a significant increase in overall yield as compared to *M*. *gryphiswaldense.* With *M*. *gryphiswaldense,* a cell yield of 1 g fresh weight per liter of culture can be typically obtained, resulting in a final yield of 10 mg magnetic nanoparticles (i.e. magnetite) per liter of culture. With *R. rubrum,* a cell yield of up to 60 mg per liter can be accomplished (by means of high cell-density fermentation). However, in *R. rubrum,* the relative amount of magnetite per cell mass can be estimated to be only about 20% of that in *M. gryphiswaldense.* Nevertheless, due to the much higher cell density of *R. rubrum,* a final yield of 100-120 mg magnetic nanoparticles (i.e. magnetite) per liter of culture can be calculated, that is, an increase by a factor of 10-12 as compared to *M*. *gryphiswaldense.*

In a further analysis, a recombinant *M*. *gryphiswaldense* strain harboring, in addition to its endogenous copies of the *mamAB, mamGFDC, mms6 ,and mamXY operons,* additional (i.e. genetically introduced) copies of the respective four expression cassettes, produced about two times the amount of magnetic nanoparticles than the corresponding wild-type strain (data not shown), thus suggesting the "gene copy number" as a further important determinant for improving the overall yield of magnetic nanoparticles produced.

### 3. Conclusions

So far, significant efforts have been made to genetically or metabolically magnetize prokaryotic and eukaryotic organisms/cells (Vainshtein, M. et al. (2002). Biol Cell 94, 29-35; Nishida, K. and Silver, P.A. (2012) PLoS Biol 10, e1001269). However, engineering resulted in only few undefined, delocalized and poorly crystalline Fe deposits.

This has prompted ideas to transfer genetic parts/the entire pathway for producing magnetic nanoparticle from magnetotactic bacteria in other organisms. Successful transfer and heterologous expression has been demonstrated for other bacterial gene clusters encoding mostly soluble metabolic pathways such as nitrogen fixation (Temme, K., et al. (2012). Proc. Natl. Acad. Sci. USA 109, 7085-7090) or synthesis of secondary metabolites (Fischbach, M. and Voigt, C.A. (2010) Biotechnol. J. 5, 1277-1290). Furthermore, the ability to synthesize structures, such as microcompartments and gas vesicles by the transfer of few genes has also been shown in foreign hosts (Li, N. and Cannon, M. C. (1998) J. Bacteriol 180, 2450-2458 (1998) Bonacci, W. et al. (2012) Proc Natl Acad Sci U S A 109, 478-483).

However, transfer and reconstitution of a structurally and genetically similarly complex gene pathways or organelles, which requires the balanced expression of a multitude of structural and catalytic membrane-bound factors has not yet been achieved. The present findings are important for a number of reasons. First, they demonstrate that the 30 genes derived from the *mamAB, mamXY, mamGFDC* and mms6 operons constitute a minimal gene set which supports the biomineralization and assembly of magnetosome chains in heterologous host cells, but auxilliary functions encoded outside the genomic MAI are required for magnetosome formation as well, although the *mamAB* operon alone has been shown to be sufficient for some rudimentary biomineralization in *M*. gryphiswaldense (Lohsse, A. *et al.* (2011) *supra).*

The distribution of similar MAI-like gene clusters in all the phylogenetically diverse magnetotactic bacteria analyzed stimulated two different hypotheses: horizontal gene transfer vs. vertical inheritance from a magnetic ancestor. However, the inheritance of magnetosome genes has remained unsolved so far. The present results provide first experimental evidence that the gene pathway for the production of magnetosomes can be transferred horizontally to a phototrophic organism and thus pointing towards a possible evolutionary link between magnetosome formation and phototrophy.

Functional reconstitution of the magnetosome pathway will provide for an analysis of gene functions by "surrogate genetics" in genetically more amenable host cells, thus resulting in an improved/and sustainable production of magnetic nanoparticles as compared to the homologous system. Furthermore, the present findings might also enable synthetic biology approaches for production of tailored magnetic nanoparticles in both prokaryotic and eukaryotic organisms, for example, for the *in vivo* synthesis of magnetic nanoparticles for applications in "magnetogenetics" such as magneto-thermal and magneto-mechanical stimulation of ion channels, or for the endogenous expression of a magnetic reporter for biomedical imaging (e.g., MRI).

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

**TABLE 1: Summary of magnetic responses (C_{mag}), intracellular iron content and crystal size and number of various strains (median values, ± = standard deviation). If not indicated otherwise, cells were grown in the presence of 50 µM ferric citrate. Magnetic response and total iron content were measured with (n) biological replicates under identical conditions. For determination of crystal size and number per cell, (n) cells of one clone were analyzed by TEM. The Mann-Whitney test (http://elegans.som.vcu.edu/~leon/stats/ utest.html) was performed for crystal size comparison of R. rubrum_ABG6X and R. rubrum_ABG6X_feo: the difference was highly significant (p < 0.001, two tailed test). Crystal size comparison of R. rubrum_ABG6X_feo and M. gryphiswaldense revealed no significant difference (p ≥ 0.05, two tailed test).**

| **Strain** | **(C_{mag})** | **Iron content (% dry weight)** | **Crystal size (nm)** | **Crystal number per cell** |
|---|---|---|---|---|
| *M. gryphiswaldense* MSR-1 | 1.4 ± 0.2 (n=3) | 3.5 (n=3) | 36 ± 9 (n = 310) | 24 ± 8 (n = 52) |
| *M. gryphiswaldense ΔmamAB_AB* | 1.2 ± 0.2 (n=3) | n.d. | 37 ± 10 (n = 112) | 23 ± 7 (n = 24) |
| *M. gryphiswaldense* MSR-1B_*AB* | 0.2 (n=3) | n.d. | 17 ± 6 (n = 112) | 16 ± 6 (n = 20) |
| *M. gryphiswaldense* MSR-1B*_ABG* | 0.6 ± 0.1 (n=3) | n.d. | 25 ± 6 (n = 104) | 13 ± 6 (n = 20) |
| *M. gryphiswaldense* MSR-1B*-ABG6* | 0.9 ± 0.2 (n=3) | n.d. | 35 ± 8 (n = 103) | 18 ± 8 (n = 22) |
| *R. rubrum* ATCC 11170 | - | 0.07 ± 0.04 (n=3) | - | - |
| *R. rubrum_AB* | - | 0.08 (n=3) | - | - |
| *R. rubrum_ABG* | - | 0.10 ± 0.01 (n = 3) | - | - |
| *R. rubrum_ABG6* | - | 0.17 (n = 4) | 12 ± 6 (n = 304) | 26 ± 10 (n = 50) |
| *R. rubrum_ABG6X* | 0.3 ± 0.2 (n=3) | 0.17 ± 0.02 (n = 4) | 24 ± 7 (n = 307) | 10 ± 4 (n = 50) |
| *R. rubrum_ABG6X* 500 µM ferric citrate | 0.3 (n = 4) | n. d. | 25 ± 7 (n = 301) | 11 ± 5 (n=51) |
| *R. rubrum_ABG6X* 100 µM ferrous sulfate | 0.2 (n = 4) | n.d. | 24 ± 8 (n = 312) | 10 ± 5 (n = 52) |
| *R. rubrum_ABG6X_ftsZm* | 0.6 ± 0.1* (n=3) | 0.18 ± 0.03 (n=3) | 26 ± 9 (n = 300) | 11 ± 4 (n=51) |
| *R. rubrum_ABG6X_dJ* | 0.2 (n=3) | 0.18 ± 0.01 (n=3) | 27 ± 9 (n=300) | 9 ± 4 (n = 50)** |
| *R. rubrum_ABG6X_dl* | - | 0.09 ± 0.07 (n = 3) | - | - |
| *R. rubrum_ABG6X_feo* | 0.8 ± 0.1 (n=3) | 0.28 ± 0.07 (n=3) | 37 ± 10 (n = 300) | 10 ± 4 (n = 52) |

| | | | | |
|---|---|---|---|---|
| *The slightly increased C_{mag} is likely due to effects of the genuine cell division protein FtsZm on cell morphology, as no difference in iron content and crystal size or number per cell was detectable. **64% of mutant cells (n = 32) harbored clustered magnetosomes, whereas 36% still showed a chain-like alignment of magnetosomes (n = 18). | | | | |

**TABLE 2: Results of BLAST analysis of 16S rRNA gene of M. gryphiswaldense against R. rubrum and potential production strains. Organisms were selected based on their close phylogenetic affiliation, availability of a genetic system or predominant role in heterologous protein expression.**

| **Organism** | **Phylogenetic class** | **16S rRNA gene identity (%)** | **NCBI Accession number** |
|---|---|---|---|
| *Rhodospirillum rubrum* | *Alphaproteobacteria* | 90 | NR_074249 |
| *Caulobacter crescentus* | *Alphaproteobacteria* | 88 | AE005673 |
| *Rhodobacter sphaeroides* | *Alphaproteobacteria* | 87 | CP000144 |
| *Rhodobacter capsulatus* | *Alphaproteobacteria* | 86 | NR_102927 |
| *Agrobacterium tumefaciens* | *Alphaproteobacteria* | 88 | AJ012209 |
| *Escherichia coli* | *Gammaproteobacteria* | 83 | U00096 |

**TABLE 3: Magnetosome proteins identified in the MM of strain R. rubrum_ABG6X by nano-electrospray ionization-LC tandem MS (ESI-LC-MS/MS). Spectra were analyzed via Mascot™ software using the NCBI nr Protein Database and a database from M. gryphiswaldense (asterisks; Richter, M. et al. (2007) J. Bacteriol. 189, 4899-4910). Proteins are listed in the order of their exponentially modified protein abundance index (emPAI). The data have been deposited to ProteomeXchange with identifier PXD000348 (DOI 10.6019/PXD000348).**

| **Protein** | **Acc. No.** | **Coverage (%)** | **No. of spectrum matches** | **No. of seq. peptides** | **MW (kDa)** | **emPAI** | **Putative function** |
|---|---|---|---|---|---|---|---|
| MamK | MGR_4093 | 57 | 9 | 9 | 39.6 | 1.51 | Magnetosome chain assembly/positioning |
| MamC | MGR_4078 | 32 | 4 | 3 | 12.4 | 1.01 | Crystal size and shape control |
| MamJ | MGR_4092 | 32 | 10 | 6 | 48.6 | 0.76 | Magnetosome chain assembly |
| MamA | MGR_4099 | 37 | 1 | 1 | 23.9 | 0.65 | TPR-like protein assoc. with the MM |
| MamF | MGR_4076 | 17 | 1 | 1 | 12.4 | 0.60 | Magnetosome size and shape control |
| Mms6 | MGR_4073 | 19 | 1 | 1 | 12.7 | 0.58 | Magnetosome crystallization |
| MamD | MGR_4077 | 20 | 3 | 3 | 30.2 | 0.49 | Crystal size and shape control |
| MamM* | MGR_4095 | 15 | 3 | 3 | 34.7 | 0.42 | Iron transport/MM assembly |
| MmsF* | MGR_4072 | 8 | 2 | 1 | 13.9 | 0.23 | Crystal size and shape control |
| MamB* | MGR_4102 | 7 | 1 | 1 | 32.1 | 0.21 | Iron transport/MM assembly |
| MamY* | MGR_4150 | 18 | 2 | 2 | 40.9 | 0.16 | Tubulation and MM formation |
| MamO* | MGR_4097 | 6 | 3 | 3 | 65.3 | 0.15 | Magnetosome crystallization |
| MamE | MGR_4091 | 4 | 2 | 2 | 78.3 | 0.08 | Magnetosome crystallization |

**TABLE 4: Strains and plasmids used in this study. Km^{R} = kanamycin resistance, Tc^{R} = tetracycline resistance, Ap^{R} = ampicillin resistance, BSD^{R} = blasticidin S resistance, Cm^{R} = chloramphenicol resistance, Gm^{R} = gentamicin resistance, Spec^{R}=spectinomycin resistance.**

| **Strain or plasmid** | **Characteristics** | **Reference or source** |
|---|---|---|
| *M. gryphiswaldense* MSR-1 | Wild-type (wt) | DSM-6361; Schleifer, K. et al. (1991) Syst. |
| *M. gryphiswaldense* MSR-1B | spontaneous unmagnetic mutant lacking parts of the MAI | Schübbe, S. et al. (2993) J. Bacteriol. 185, 5779-5790 |
| *M. gryphiswaldense ΔmamAB* | *mamAB* deletion mutant | Lohsse, A. et al. (2011) PLoS One 6, e25561 |
| *M. gryphiswaldense ΔmamAB _AB* | Km^{R}, transposon mutant with inserted *mamAB* operon | This study |
| *M. gryphiswaldense* MSR-1B_AB | Km^{R}, transposon mutant with inserted *mamAB* operon | This study |
| *M. gryphiswaldense* MSR-1B_ABG | Km^{R}, Spec^{R}, transposon mutant with inserted *mamAB and mamGFDC* operon | This study |
| *M. gryphiswaldense* MSR-1B_ABG6 | Km^{R}, Cm^{R}, transposon mutant with inserted *mamAB, mamGFDC* and mms6 operon | This study |
| *R. rubrum* ATCC 11170 | Wild-type (wt) | Pfenning, N. & Trüper H.G. (1971) Int.J. Syst. Bacteriol. 21, 19-24 |
| *R. rubrum_AB* | Km^{R}, transposon mutant with inserted *mamAB* operon | This study |
| *R. rubrum_ABG* | Km^{R}, Spec^{R}, transposon mutant with inserted *mamAB and mamGFDC* operon | This study |
| *R. rubrum_ABG6* | Km^{R}, Cm^{R}, transposon mutant with inserted *mamAB, mamGFDC* and mms6 operon | This study |
| *R. rubrum_ABG6X* | Km^{R}, Cm^{R}, Gm^{R} transposon mutant with inserted *mamAB, mamGFDC,* mms6 and *mamXY* operon (without *ftsZm)* | This study |
| *R. rubrum_ABG6X_dJ* | Km^{R}, Cm^{R}, Gm^{R}, Ap^{R} transposon mutant with inserted *mamAB (mamJ* deletion), *mamGFDC,* mms6 and *mamXY* operon (without *ftsZm)* | This study |
| *R. rubrum_ABG6X_dl* | Km^{R}, Cm^{R}, Gm^{R}, Ap^{R} transposon mutant with inserted *mamAB* (*mamI* deletion), *mamGFDC,* mms6 and *mamXY* operon (without *ftsZm)* | This study |
| *R. rubrum_ABG6X_ftsZm* | Km^{R}, Cm^{R}, Gm^{R}, Tc^{R} transposon mutant with inserted *mamAB, mamGFDC,* mms6 and *mamXY* operon (without *ftsZm*) and *ftsZm* under control of an inducible lac promoter | This study |
| *R. rubrum_ABG6X_feo* | Km^{R}, Cm^{R}, Gm^{R}, Tc^{R} transposon mutant with inserted with inserted *mamAB, mamGFDC,* mms6, *mamXYand feoAB1* operon | This study |
| *R.rubrum_GFDC-EGFP* | Tc^{R} transposon mutant with inserted *mamGFDC-EGFP* | This study |
| *R.rubrum_ABG6X_GFDC-EGFP* | Km^{R}, Cm^{R}, Gm^{R}, Tc^{R} transposon mutant with inserted *mamAB, mamGFDC,* mms6 and *mamXY* operon (without *ftsZm)* and *mamGFDC-EGFP* | This study |
| *R. rubrum J-EGFP* | Tc^{R} transposon mutant with inserted *mamGFDC-EGFP* | This study |
| *R. rubrum_ABG6X_J-EGFP* | Km^{R}, Cm^{R}, Gm^{R}, Tc^{R} transposon mutant with inserted *mamAB, mamGFDC,* mms6 and *mamXY* operon (without *ftsZm)* and *mamJ-EGFP* | This study |
| *E.coli* DH10b | *F- mcrA Δ(mrr-hsdRMS-mcrBC) φ80lacZΔM15 ΔlacX74 recA1 endA1 araD139 Δ(ara leu)* 7697 *galU galK rpsL nupG λ*- | Invitrogen |
| *E.coli* BW29427 | *dap* auxotroph derivative of *E. coli* strain B2155 | K. Datsenko & B. L. Wanner, unpublished |
| *E.coli* WM3064 | *thrB1004 pro thi rpsL hsdS lacZΔM15 RP4-1360 Δ(araBAD)567 ΔdapA1341::[erm pir]* | W. Metcalf, kindly provided by J. Gescher, KIT Karlsruhe |
| pSC101-BAD-gbaA | Tc^{R}, replicative plasmid containing redα/redβ recombinases under the control of a L-Arabinose inducible promoter, temperature sensitive origin of replication | Wang, J. et al. (2006) Mol. Biotechnol. 32, 43-53 |
| p15A-Tps-oriT-Km | Km^{R}, BSD^{R}, oriT, p15A origin of replication, mariner tps, cloning cassette | Fu, J. et al. (2008) Nucleic Acids Res. 36, e113 |
| pSSK18 (BAC_*mamAB*) | BAC containing the *mamAB* operon from M. *gryphiswaldense* | Schübbe, S. et al. (2993) J. Bacteriol. 185, 5779-5790 |
| pTps_AB | Km^{R}, BSD^{R}, mariner tps vector containing *mamAB* operon | This study |
| pTps_ABG | Spec^{R}, Km^{R}, BSD^{R}, mariner tps vector with *mamAB* and *mamGFDC* operon | This study |
| pTps_ABG6 | Cm^{R}, Km^{R}, BSD^{R}, mariner tps vector with *mamAB, mamGFDC,* and mms6 operon | This study |
| pTps_XYZ | Gm^{R}, BSD^{R}, mariner Tps vector with *mamY, mamX* and *mamZ* | This study |
| pTps_ABG6_dJ | Cm^{R}, Km^{R}, BSD^{R}, Ap^{R}, mariner tps vector with *mamAB, mamGFDC,* and mms6 operon, (*mamJ* deletion) | This study |
| pTps_ABG6_dl | Cm^{R}, Km^{R}, BSD^{R}, Ap^{R}, mariner tps vector with *mamAB, mamGFDC,* and mms6 operon, (*mamI* deletion) | This study |
| pBAM1 | Km^{R}, Ap^{R}, γR6K origin of replication, oriT, Tn5 vector | Martinez-Garcia, E. et al. (2011) BMC Microbiol. 11, 38 |
| Tet-pBAM1 | Tc^{R}, Ap^{R}, γR6K origin of replication, oriT, Tn5 vector | This study |
| pBam_mamGFDC-EGFP | Tc^{R}, Ap^{R}, *mamGFDC* operon under control of P*_{mamDC}* with a C-terminal EGFP fusion, Tn5 vector | This study |
| pBam_MamJ-EGFP | Tc^{R}, Ap^{R}, *mamJ* under control of *P_{mamDC}* with a C-terminal EGFP fusion, Tn5 vector | This study |
| pRU-1feoAB | Km^{R}, broad host range pBBRMCS2, *feoAB1* operon under the control of P*_{mamH}* | R. Uebe, unpublished |
| pBam-feoAB1 | Tc^{R}, Ap^{R}, *feoAB1* operon under the control of P*_{mamH},* Tn5 vector | This study |
| pBam-ftsZm_mCherry | Tc^{R}, Ap^{R}, *ftsZm, lacl* with a C-terminal mcherry fusion under control of inducible P*_{lac}*, Tn5 vector | This study |
| pFM211 | Km^{R}, broad host range pBBRMCS2, lacI, *ftsZm* with C-terminal *mCherry* fusion, *mamK* with N-terminal EGFP fusion | F. Müller, unpublished |

**TABLE 5: Oligonucleotides used in this study.**

| **SEQ ID NO:** | **Primer** | **Nucleotide sequence (5'-3')^{a}** | **Product** |
|---|---|---|---|
| 1 | Mam-tps5 | AATTCGCACGGACTATAGCAACGAATCGAGGTCG GTTGACAAGCCATAAATCAGAAGAACTCGTCAAGA AGGC | p15A-Tps-oriT-Km, ET-recombination with *BAC_mamAB,* pTps_AB |
| 2 | Mam-tps3 | GAACGAAGATGAGACAGAAATCCGTGGCGCCGAG CGTAAGCATCCGGTGAGAACCTCATTCCCTCATGA TACAG | |
| 3 | mamGFC3 | TATCATGAGGGAATGAGGTTCTCACCGGATGCTTA CGCTCGGCGCCAGAGCACATCGGGGTGAATGAC GAC | *mamGFDC* operon, ET-recombination with pTps_AB |
| 4 | mamGFC5 | CGCTAGCTGCGGGTTATTCGCATTTGC | |
| 5 | spectMam3 | TCAAAACCCGCGCAGAGGCAAATGCGAATAACCC GCAGCTAGCGTTATAATTTTTTTAATCTGTTATT | Spectinomycin resistance cassette, ET-recombination with pTps_AB |
| 6 | spectMam5 | TGATCCGCTATGGTAAGCGCATCATGTCCGGATCC CATGGCGTTCCGCTCGTAACGTGACTGGCAAGAG ATATT | |
| 7 | mms6cm5 | TACTGCGATGAGTGGCAGGGCGGGGCGTAAGCTT ACAATTTCCATTCGCCATTC | mms6 operon, ET-recombination with pTps_ABG |
| 8 | mms6mam3 | GTGCTTCGCTGTGTCCACAAGAACC | |
| 9 | cm-mms6-3 | TGGCGAATGGAAATTGTAAGCTTACGCCCCGCCC TGCCACTC | Chloramphenicol resistance cassette, ET-recombination with pTps_ABG |
| 10 | cm-mms6-5 | TGATCCGCTATGGTAAGCGCATCATGTCCGGATCC CATGGCGTTCCGCTCGTCCTGGTGTCCCTGTTGAT ACC | |
| 11 | IK097 | TCTAGAGGGCCCCAACTTTTTCGCTTTACTAGCTC TTAGTTCTCCAATAAATTCCCTGCGTCGA | P*_{mamDC}* in pBAM1 |
| 12 | IK098 | CATATGCTGATCTCCGGCAAGTGTATGCACGATTC CCTCTCTGCCCCTTAAAATCGACGCAGGGAAT | |
| 13 | IK107 | CATATGATCAAGGGCATCGCGGG | *mamGFDC* operon in pBAM1 |
| 14 | IK101 | GGTACCGGCCAATTCTTCCCTCAGAA | |
| 15 | IK102 | GGTACCGGAGGCGGAGGCGGT | *egfp* in pBAM1 |
| 16 | IK103 | GAATTCTTACTTGTACAGCTCGTCCATG | |
| 17 | IK163 | GAATTCTTAGCCGATTCGCAG | *mamXY*-operon (without ftsZm), ET-recombination with p15A-Tps-oriT-Gm |
| 18 | IK164 | GAGCTCGGCAGCCTCATTTAAA | |
| 19 | IK173 | CCGGAATTGCCAGCTGGGGCGCCCTCTGGTAAGG TTGGGAAGCCCTGCAACGTATAATATTTGCCCATG | Gentamicin resistance cassette, ET-Recombination with p15A-Tps-oriT-Km |
| 20 | IK174 | AGGCGATAGAAGGCGATGCGCTGCGAATCGGGA GCGGCGATACCGTAAAGCGATCTCGGCTTGAA | |
| 21 | IK208 | CCCGGTACCCAGCTTTTGTTCCCTTTAGTGAGGGT TAATTGCGCGCTTGGCCTCATTCCCTCATGATACA GAGAC | p15A-Tps-oriT-Gm, ET-recombination with *mamXY* |
| 22 | IK209 | GGCGTTACCCAACTTAATCGCCTTGCAGCACATCC CCCTTTCGCCAGCTGTCTCGGCTTGAACGAATTG | |
| 23 | IK213 | GACGTCGAGCCACGGCGG | Tetracycline resistance cassette in pBAM1 |
| 24 | IK214 | GGGTCCCTCAGGTCGAGGTGGC | |
| 25 | IK215 | TCTAGACTACAAGAATGTCCCGC | feoABI operon+P*_{mamH}* in pBAM1 |
| 26 | IK216 | GAATTCGGCATCCTGATCGGT | |
| 27 | IK217 | CATATGATGGCAAAAAACCGG | *mamJ* in pBAM1 |
| 28 | IK218 | GGCGGTACCTTTATTCTTATCTTCAGCATCAC | |
| 29 | IK235 | GGGTGGAGCGGGATAATGGCAAAAAACCGGCGTG ATCGCGGCACGGCTAAATACATTCAAATATGTATC C | Ampicillin resistance cassette insertion into *mamJ* of pTps_ABG6 |
| 30 | IK236 | CTATTTATTCTTATCTTCAGCATCACATTTCGGCGA TGAACAACTACCTTACCAATGCTTAATCAGTG | |
| 31 | IK239 | CGCCGCTTGTGTTCTGTATCAAGACTGGAGAACGT TTATGCCAACTAAATACATTCAAATATGTATCC | Ampicillin resistance cassette Insertion into *mamI* of pTps_ABG6 |
| 32 | IK240 | TCAACCATCGATGTTAGGGTCTGAGTTCGCCCTCT TACCGGCAGGTTACCAATGCTTAATCAGTG | |
| 33 | IK251 | AAACCGCCCAGTCTAGCTATCGCCATGTAAGCCCA CTGCAAGCTACCTGCCCTCATTCCCTCATGATACA | Tet-pBam1, ET-recombination with pFM211 |
| 34 | IK252 | CAGCACATCCCCCTTTCGCCAGCTGGCGTAATAG CGAAGAGGCCCGCACCGGATTTTGAGACACAAGA CGTC | |

## Claims

1. Recombinant host cell, comprising in its genome:
(i) a gene expression cassette capable of being expressed in the host cell, the gene expression cassette encoding a nucleic acid sequence encompassing at least 80% of the full-length sequence of the *mamAB* operon of a magnetotactic alphaproteobacterium;
(ii) a gene expression cassette capable of being expressed in the host cell, the gene expression cassette encoding a nucleic acid sequence encompassing at least 80% of the full-length sequence of the *mamGDFC* operon of a magnetotactic alphaproteobacterium; and
(iii) a gene expression cassette capable of being expressed in the host cell, the gene expression cassette encoding a nucleic acid sequence encompassing at least 80% of the full-length sequence of the *mms6* operon of a magnetotactic alphaproteobacterium;
wherein the recombinant host cell, upon expression of the gene expression cassettes in their entirety, is capable of producing magnetic nanoparticles.

2. The recombinant host cell of claim 1, further comprising:
(iv) a gene expression cassette capable of being expressed in the host cell, the gene expression cassette encoding a nucleic acid sequence encompassing at least 80% of the full-length sequence of the *mamXY* operon of a magnetotactic alphaproteobacterium; and/or
(v) a gene expression cassette capable of being expressed in the host cell, the gene expression cassette encoding a nucleic acid sequence encompassing at least 80% of the full-length sequence of the *feoAB1* operon of a magnetotactic alphaproteobacterium.

3. The recombinant host cell of claim 2, comprising gene expression cassettes (i) to (v), wherein the gene expression cassettes all encompass the full-length sequences of the respective operons.

4. The recombinant host cell of claim 3, wherein the total length of the gene expression cassettes comprised in the genome is less than 35 kb.

5. The recombinant host cell of any one of claims 1 to 3, wherein the magnetotactic alphaproteobacterium is *Magnetospirillum spec.,* and in particular *Magnetospirillum gryphiswaldense.*

6. The recombinant host cell of any one of claims 1 to 5, wherein the gene expression cassettes are stably integrated into the host cell's genome.

7. The recombinant host cell of any one of claims 1 to 6, wherein the magnetic nanoparticles are magnetosomes, and in particular magnetosomes consisting of magnetite.

8. The recombinant host cell of any one of claims 1 to 7, wherein any one or more of the gene expression cassettes are under the control of their respective endogenous regulatory sequences.

9. The recombinant host cell of any one of claims 1 to 8, wherein the host cell comprises two or more copies of any one or more of the gene expression cassettes.

10. The recombinant host cell of any one of claims 1 to 9, wherein any one or more of the gene expression cassettes represent heterologous nucleic acid sequences.

11. The recombinant host cell of any one of claims 1 to 10, wherein the host cell is a prokaryotic cell, and particularly derived from an alphaproteobacterium.

12. The recombinant host cell of claim 11, wherein the host cell is derived from *Rhodospirillum rubrum.*

13. Method for the production of a recombinant host cell as defined in any one of claims 1 to 12, the method comprising the transfer of the gene expression cassettes into the host cell by means of genetic transposition, and in particular comprising a modular transfer of the gene expression cassettes.

14. Use of a recombinant host cell as defined in any one of claims 1 to 12 for the biotechnological production of magnetic nanoparticles.

15. Use of a recombinant host cell as defined in any one of claims 1 to 12 for the *in vivo* synthesis of magnetic nanoparticles for application in magnetogenetics or biomedical imaging.


SEQUENCE LISTING

<110>   Ludwig-Maximilians-Universit\204t M\201nchen

<120>   Production of magnetic nanoparticles in recombinant host cells

<130>   835-9

<140>   not assigned yet
<141>   2013-11-18

<160>   37

<170>   BiSSAP Version 1.1

<210>   1
<211>   73
<212>   DNA
<213>   artificial sequence

<220>
<223>   oligonucleotide primer Man-tps5

<400>   1

aattcgcacg gactatagca acgaatcgag gtcggttgac aagccataaa tcagaagaac        60
tcgtcaagaa ggc                                                           73

<210>   2
<211>   74
<212>   DNA
<213>   artificial sequence

<220>
<223>   oligonucleotide primer Man-tps3

<400>   2

gaacgaagat gagacagaaa tccgtggcgc cgagcgtaag catccggtga gaacctcatt        60
ccctcatgat acag                                                          74

<210>   3
<211>   71
<212>   DNA
<213>   artificial sequence

<220>
<223>   oligonucleotide primer mamGFC3

<400>   3

tatcatgagg gaatgaggtt ctcaccggat gcttacgctc ggcgccagag cacatcgggg        60
tgaatgacga c                                                             71

<210>   4
<211>   27
<212>   DNA
<213>   artificial sequence

<220>
<223>   oligonucleotide primer mamGFC5

<400>   4

cgctagctgc gggttattcg catttgc                                          27

<210>   5
<211>   68
<212>   DNA
<213>   artificial sequence

<220>
<223>   oligonucleotide primer spectMam3

<400>   5

tcaaaacccg cgcagaggca aatgcgaata acccgcagct agcgttataa tttttttaat      60
ctgttatt                                                               68

<210>   6
<211>   74
<212>   DNA
<213>   artificial sequence

<220>
<223>   oligonucleotide primer spectMam5

<400>   6

tgatccgcta tggtaagcgc atcatgtccg gatcccatgg cgttccgctc gtaacgtgac      60
tggcaagaga tatt                                                        74

<210>   7
<211>   54
<212>   DNA
<213>   artificial sequence

<220>
<223>   oligonucleotide primer mms6cm5

<400>   7

tactgcgatg agtggcaggg cggggcgtaa gcttacaatt tccattcgaa attc            54

<210>   8
<211>   25
<212>   DNA
<213>   artificial sequence mms6mam3

<220>
<223>   oligonucleotide primer mms6mam3

<400>   8

gtgcttcgct gtgtccacaa gaacc                                            25

<210>   9
<211>   42
<212>   DNA
<213>   artificial sequence

<220>
<223>   oligonucleotide primer cm-mms6-3

<400>   9

tggcgaatgg aaattgtaag cttacgcccc gccctgccac tc                42

<210>   10
<211>   73
<212>   DNA
<213>   artificial sequence

<220>
<223>   oligonucleotide primer cm-mms6-5

<400>   10

tgatccgcta tggtaagcgc atcatgtccg gatcccatgg cgttccgctc gtcctggtgt    60
ccctgttgat acc                                                       73

<210>   11
<211>   64
<212>   DNA
<213>   artificial sequence

<220>
<223>   oligonucleotide primer IK097

<400>   11

tctagagggc cccaactttt tcgctttact agctcttagt tctccaataa attccctgcg    60
tcga                                                                 64

<210>   12
<211>   67
<212>   DNA
<213>   artificial sequence

<220>
<223>   oligonucleotide primer IK098

<400>   12

catatgctga tctccggcaa gtgtatgcac gattccctct ctgcccctta aaatcgacgc    60
agggaat                                                              67

<210>   13
<211>   23
<212>   DNA
<213>   artificial sequence

<220>
<223>   oligonucleotide primer IK107

<400>   13

catatgatca agggcatcgc ggg                                            23

<210>   14
<211>   26
<212>   DNA
<213>   artificial sequence

<220>
<223>   oligonucleotide primer IK101

<400>   14

ggtaccggcc aattcttccc tcagaa                                          26

<210>   15
<211>   21
<212>   DNA
<213>   artificial sequence

<220>
<223>   oligonucleotide primer IK102

<400>   15

ggtaccggag gcggaggcgg t                                               21

<210>   16
<211>   28
<212>   DNA
<213>   artificial sequence

<220>
<223>   oligonucleotide primer IK103

<400>   16

gaattcttac ttgtacagct cgtccatg                                        28

<210>   17
<211>   28
<212>   DNA
<213>   artificial sequence

<220>
<223>   oligonucleotide primer IK163

<400>   17

gaattcttag ttgtacagct cgtccatg                                        28

<210>   18
<211>   22
<212>   DNA
<213>   artificial sequence

<220>
<223>   oligonucleotide primer IK164

<400>   18

gagctcggca gcctcattta aa                                              22

<210>   19
<211>   69
<212>   DNA
<213>   artificial sequence

<220>
<223>   oligonucleotide primer IK173

<400>   19

ccggaattgc cagctggggc gccctctggt aaggttggga agccctgcaa cgtataatat     60
ttgcccatg                                                             69

<210>   20
<211>   65
<212>   DNA
<213>   artificial sequence

<220>
<223>   oligonucleotide primer IK174

<400>   20

aggcgataga aggcgatgcg ctgcgaatcg ggagcggcga taccgtaaag cgatctcggc      60
ttgaa                                                                  65

<210>   21
<211>   75
<212>   DNA
<213>   artificial sequence

<220>
<223>   oligonucleotide primer IK208

<400>   21

cccggtaccc agcttttgtt ccctttagtg agggttaatt gcgcgcttgg cctcattccc      60
tcatgataca gagac                                                       75

<210>   22
<211>   69
<212>   DNA
<213>   artificial sequence

<220>
<223>   oligonucleotide primer IK209

<400>   22

ggcgttaccc aacttaatcg ccttgcagca catccccctt tcgccagctg tctcggcttg      60
aacgaattg                                                              69

<210>   23
<211>   18
<212>   DNA
<213>   artificial sequence

<220>
<223>   oligonucleotide primer IK213

<400>   23

gacgtcgagc cacggcgg                                                    18

<210>   24
<211>   22
<212>   DNA
<213>   artificial sequence

<220>
<223>   oligonucleotide primer IK214

<400>   24

gggtccctca ggtcgaggtg gc                                               22

<210>   25
<211>   23
<212>   DNA
<213>   artificial sequence

<220>
<223>   oligonucleotide primer IK215

<400>   25

tctagactac aagaatgtcc cgc                                               23

<210>   26
<211>   21
<212>   DNA
<213>   artificial sequence

<220>
<223>   oligonucleotide primer IK216

<400>   26

gaattcggca tcctgatcgg t                                                 21

<210>   27
<211>   21
<212>   DNA
<213>   artificial sequence

<220>
<223>   oligonucleotide primer IK217

<400>   27
catatgatgg caaaaaaccg g                                                 21

<210>   28
<211>   32
<212>   DNA
<213>   artificial sequence

<220>
<223>   oligonucleotide primer IK218

<400>   28

ggcggtacct ttattcttat cttcagcatc ac                                     32

<210>   29
<211>   70
<212>   DNA
<213>   artificial sequence

<220>
<223>   oligonucleotide primer IK235

<400>   29

gggtggagcg ggataatggc aaaaaaccgg cgtgatcgcg gcacggctaa atacattcaa      60
atatgtatcc                                                              70

<210>   30
<211>   68
<212>   DNA

<213>   artificial sequence

<220>
<223>   oligonucleotide primer IK236

<400>   30

ctatttattc ttatcttcag catcacattt cggcgatgaa caactacctt accaatgctt      60
aatcagtg                                                                68

<210>   31
<211>   68
<212>   DNA
<213>   artificial sequence

<220>
<223>   oligonucleotide primer IK239

<400>   31

cgccgcttgt gttctgtatc aagactggag aacgtttatg ccaactaaat acattcaaat      60
atgtatcc                                                                68

<210>   32
<211>   65
<212>   DNA
<213>   artificial sequence

<220>
<223>   oligonucleotide primer IK240

<400>   32

tcaaccatcg atgttagggt ctgagttcgc cctcttaccg gcaggttacc aatgcttaat      60
cagtg                                                                   65

<210>   33
<211>   70
<212>   DNA
<213>   artificial sequence

<220>
<223>   oligonucleotide primer IK251

<400>   33

aaaccgccca gtctagctat cgccatgtaa gcccactgca agctacctgc cctcattccc      60
tcatgataca                                                              70

<210>   34
<211>   72
<212>   DNA
<213>   artificial sequence

<220>
<223>   oligonucleotide primer IK252

<400>   34

cagcacatcc ccctttcgcc agctggcgta atagcgaaga ggcccgcacc ggattttgag      60
acacaagacg tc                                                           72

<210>   35

<211>   26564
<212>   DNA
<213>   artificial sequence

<220>
<223>   gene expression cassette comprising the mamAB, mamGDFC, and mms6
operons of Magnetosprillum gryphiswaldense

<400>   35

acaggttggc tgataagtcc ccggtcttca cgctgccgca agcactcagg gcgcaagggc      60
tgctaaagga agcggaacac gtagaaagcc agtccgcaga ggcggtgctg accccggatg     120
aatgtcagct actgggctat ctggacaagg gaaaacgcaa gcgcaaagag aaagcaggta     180
gcttgcagtg ggcttacatg gcgatagcta gactgggcgg ttttatggac agcaagcgaa     240
ccggaattgc cagctggggc gccctctggt aaggttggga agccctgcaa agtaaactgg     300
atggctttct tgccgccaag gatctgatgg cgcaggggat caagatctga tcaagagaca     360
ggatgaggat cgtttcgcat gattgaacaa gatggattgc acgcaggttc tccggccgct     420
tgggtggaga ggctattcgg ctatgactgg gcacaacaga caatcggctg ctctgatgcc     480
gccgtgttcc ggctgtcaga gcaggggcgc ccggttcttt ttgtcaagac cgacctgtcc     540
ggtgccctga atgaactgca ggacgaggca gcgcggctat cgtggctggc cacgacgggc     600
gttccttgcg cagctgtgct cgacgttgtc actgaagcgg gaagggactg gctgctattg     660
ggcgaagtgc cggggcagga tctcctgtca tctcaccttg ctcctgccga gaaagtatcc     720
atcatggctg atgcaatgcg gcggctgcat acgcttgatc cggctacctg cccattcgac     780
caccaagcga aacatcgcat cgagcgagca cgtactcgga tggaagccgg tcttgtcgat     840
caggatgatc tggacgaaga gcatcagggg ctcgcgccag ccgaactgtt cgccaggctc     900
aaggcgcaca tgcccgacgg cgaggatctc gtcgtgaccc atggcgatgc ctgcttgccg     960
aatatcatgg tggaaaatgg ccgcttttct ggattcatcg actgtggccg gctgggtgtg    1020
gcggaccgct atcaggacat agcgttggct acccgtgata ttgctgaaga gcttggcggc    1080
gaatgggctg accgcttcct cgtgctttac ggtatcgccg ctcccgattc gcagcgcatc    1140
gccttctatc gccttcttga cgagttcttc tgatttatgg cttgtcaacc gacctcgatt    1200
cgttgcctat agtccgtgcg aattggaggt gaattgtgac gggaatggaa cctggcagat    1260
cagaagttga ggggcaccag cgcaacgccc tttatttatt gtcggcgttg tgcatggttt    1320
tcatgactct cgtcgttgct attcagccgc tttttttgcg aaacgttctt aatatcccct    1380
tcgagactgc cggggcagtc aatgccaatg tgcaggtggt gaccgaggtc cttgatcttt    1440
tcatctttgc ctatcttggt tacctgtcag atcgcattgg ccgggtccgg atcatcgtgg    1500
cgggctttct cgtcgctgcg gtcggcgctg tgatcgcgcc gctaagtccc tgggttggcg    1560
gagcctccat tggggcgctg gtggtctact acgtttcgcg ggtcatcatg tctgcgggca    1620
gcggtgctgt gtggccgcaa ttgtcggccc tggccggtga tttcagtgac gacagcaacc    1680
gggctcggct gctgtccaat accgccttta tgatggcgtt cggcgtgacg ctggtctatg    1740
cggtcctgat gcagatccct ggccatgcgg gtattacggt gaccatgttg ctgactgcgg    1800
ccatttccgt ggccggtgct tggctggcgc gcaaatttct cgtggatgtg gctccgcgca    1860
ccgtcgagac ctctgtcccc tggcgggccg tttgggatct ggtgaaggcg gagccgcgcc    1920
tgcggctggc ctttgcaagt tcattgttcg ctcgaagcga tatggtgttt gtcggactgt    1980
tcctcatgct gtggttcatc tacttcgccg acctcgtaaa ggtcgggcag gcggaggcgg    2040
ctgcaagggc gggcatgttg atcggattga tgggggcggt ggtcatgctc tcgattccgg    2100
tctggcgctc gtttatcgag cgtttcgggc gcattcaggc cgttttattg gggatgatgc     2160
tatccgccct tggatttata atgctcggat ttgtggtcaa tccatttgac ggatttattg    2220
tgttttccat tctgttggtt tcggccgggc aagctggttg ctttgttgct ccgcaaatac    2280
taaccgttga tcatgcacca aaagacttgc tcggttctgt tctcggcgca tttaatgtta    2340
ttgggtgcct gggaattatt ttttttgttc agattggtgg gtttttattt gattacattg    2400
gccctcctgc gccttttatc tttacgggcg ttggaaattt gattatatca gcatatgcgt    2460
tgcgtcttgt gaaaagtgag gcgcgtgggg ttggcggggg taacacccca ggggatgacc    2520
aagtggccta gcgccgcttg tgttctgtat caagactgga gaacgtttat gccaagcgtg    2580
attttcgggc tgctggcgct tgccattgga ttgctggggc tgacggcgtg gtggtggtcg    2640
gtgaccgagt ttctgcgtgg tgcggtgccg gtggctctga tcatcttcgg cttggttgcg    2700
ctggccgcag gggtccagtc ggtgcgagtg cctcctgccg gtaagagggc gaactcagac     2760
cctaacatcg atggttgatg accatgttca atggtgatgt ggaagacggt ggccgttcca    2820
atgtctcttg cggcaaagac ctgaagcgct acctgatgct catgggcgtt gtcgccctgg    2880
tcgtcctgtt cggcgcgttc atctatcggc agtcctcggg cggtcttcgc ctgggcgcca    2940
tgatggagca gatgacaggc gcacgcggcg cagtgaacgt tcctgcgcag catggcgcgc    3000
cgtcggcggt ggtcgatccg gcgatgtcgg ttccggctag ggcgcgcgtg gcgccgccct    3060
cggctgccgg tgctatcgcg accttccctc cggtggtgga ttttggaccg gccccggtgg    3120
tgagcggtgg cccgttcacc ggcgtcgtaa cccttctgcg caatagcgtg gttagcgtga    3180

ccgcctcctc atccggcggc caagtcatgc ccgatccctt ggggttggtc aatcccgacg 3240
gtcttccccg tttcgccaat cccaccactc ggtcggtgga gaatatcggc accggcgtta 3300
tcgtccgcaa tgacggcttc atcgtcacca attaccatgt ggtgcgcggg gccaattcgg 3360
tgtacgtcac cgtaaaggac gatgtgggtt cgatccgcta ttccggcgag atcgtcaaga 3420
tggatgaggc gctggatctc gctttgctca agatcacccc caaggtccag cttaccgctg 3480
cggttctggg cgacagcgat gcggtgaacg tcgctgacga ggtgatcgcc atcgggactc 3540
ccttcggcct ggatatgacc gtcagccgcg gcatcatttc cgccaagcgc aaaaccatgg 3600
tcatcgaagg gatgacgcac tccaacctgc tgcagaccga cgcggcgatc aatcagggta 3660
attccggcgg tccactggtg gcggccaacg gcacggtcgt cggcatcaat acggcaatct 3720
atacccccaa tggtgcgttt gctgggatcg gctttgccgt gcccagcaat caggcccgcc 3780
ttttcgcact ggatgaagtg ggctggttgc cgacatccac cgccgagggg ccggccatgg 3840
gactggtggc catgcagcgg cccatgggtg tcggtgtcgg tgcggcaggt ccggtgattg 3900
ctgcgggtac tccgtcgccg catgtggatg gccgccagaa catggattgc agcaattgtc 3960
acgatattat tcccgccggt aatggtttcc aggcgccgat gatgcccgtg gctgctcccg 4020
tgccgccacc gccgattccc gcgaatgccg tatcacctca caccgatggt cggcaaaaca 4080
tgacctgcaa cacatgccac cagttcgtcg gtggtgcggc agccggtccg atcgcctttg 4140
gccaaccgat gatgcccatt gccgcaccgc agcaacccgc accggcgatt cgggccaatg 4200
ccgccaaccc tcacacggac ggtcggcaga acatgaactg cgccagctgc catcagatca 4260
tcggctccgt cggcgcggct cccatcgccg cgccgggagc tgggggcgcc tatcgctttt 4320
cccagccgcc agggagcctg gccatcaata tccaggggcc gcgcggtggg cagggggctg 4380
ttgccggaag tggtggtagc cgggcctcgc tcctgggggc ggctttgacc cccttgaccc 4440
agcgtctggg gctgcaggct aacctgcctg ctggtcgcgg ggtgttcgtg aacggcgtta 4500
ctcccaatac ccctgccgca tctgcggggc tgcgtcctgg cgacgttatc ctcaaagtcg 4560
atggccgtcc cgtgcatcaa cccgaggaag tggcagccat catggccgag atgcccaacg 4620
gtcgttcggt gcgaatcgga gtcctgcgtg ccggcgatgt gagcaacatg tctcttgtca 4680
ccggcccatc gggtctggcg gctgccgtgg ttcaggcacc tacggctccg gtcgtgatgg 4740
cggggggcgc tcccacggtt cctggcgttc agcctgttat tccgaaggta ccgaccgagt 4800
tcaactggct ggggatggag atcgagactt ttatggctcc gcagccggtg gtcggcatgc 4860
cgggcgctac tcctgttgcc ggggggggta aaggggctca ggtggccgag gtgttggccg 4920
gatcacgcgc cgcagtggcg ggcttcaggg ccaatgatct tatcatcgag gtaaataatc 4980
ggccggtgac aagccccgct cggcttgatg ccgccattaa ggcggcgacg gccgccgggc 5040
agcagatttt gctcaaggtc catcgcaatg gccaagagtt ctggattgtt ctttgagggt 5100
ggagcgggat aatggcaaaa aaccggcgtg atcgcggcac ggaccttcct ggcgatggcg 5160
atcaaaagat ctcgacgggg ccggagattg tttcggtgac ggtccatccc tcgccgaacc 5220
ttgctgcggc ggccaagccg gtgcagggcg acatttgggc tagcttgctt gaaagttcgc 5280
cgtggtcagc taatcaaggg gggctggtcg agacggcaca acctccttcc gcccccattc 5340
gttcccagga ccctgtgccg gttgccgatc tggtcaaccg ttggtcacag ccgatctggc 5400
gaacagcccc gctggccggg aatgcagaat cctctgagga gggcgtcgta gctccttcgc 5460
tgacgcagtc ggattctgtg cttgccgtgt ctgatctggt gattgatgtc cagccggaaa 5520
cgaatgccga ggttgaagtt tccatcgagc cggaacccgc gctggtcgag ccggtgatcg 5580
agatcgaagc cgaagccgcc gaagtcgagc cggaacctgc gccggttgcc gatctggtca 5640
accgttgggc acagccgatc tggcgaacag ccccgctggc cgggaatgca gaatcctctg 5700
aggagggcgt cgtagctcct tcgctgacgc agtcggattc tgtgcttgcc gtgtctgatc 5760
tggtgattga tgtccagccg gaagcgaatg ccgaggttga agtttccatc gagccggaac 5820
ccgcgctggt cgagccggtg atcgagatcg aagccgaagc cgccgaagtc gagccggaac 5880
ccgcgccggt tgagccggcg atcgagatcg aagccatcag ggttgaactg gaacctgtaa 5940
ttatcgacca ggtagtcgag ttggtgaccg agttcgagta ttcgcaggcg gagagcgttg 6000
catcagcgga tttgatcgct aatcccgcac ctgctgaaag ctcccgtctt gccgagcttc 6060
tggatgaggc tgctgctatt gctgctcccg ccgttgctgt ggcggtcgaa gccacccgcc 6120
agcctaacaa gatcaccgcg tcagttaaga agcgcgcccc ggttcaggaa gttcccgtgg 6180
aagaccttct gggggggatt ttcggcgtcg ccggatcggc ggtgcggggt gtgttcacta 6240
ttggcggcgg attcgtcgat ggagtggtca agggaggccg cctcgttgga agcaatgtgg 6300
tcgccgggac gcgccggttg gcgcaaacta tcgaggtaag ttgcggtagt tgttcatcgc 6360
cgaaatgtga tgctgaagat aagaataaat agagacctgt ggattgatct gttagcacgc 6420
gaacggagtg acaaaatgag tgaaggtgaa ggccaggcca agaacaggtt gttccttggc 6480
atcgaccttg ggacttccca taccgcggtg atgacgagcc ggggaaagaa gttcctgctg 6540
aagtcagtgg ttggataccc gaaggatgtc attggcttga agctgctcgg tcgcccctat 6600
gtggttggcg atgaagcctt cgagatgcgc tcctatctgg atctccgcta tccgctccag 6600
gacggtgtgc tcagcgagat cagtgaccgc gacatcgagg tggcgcgtca cctgctgacc 6720
catgtggtga agtcggcgga gccgggcgcc aacgacgaga tttgcgcggt catcggcgtg 6780
ccggcccgag cgtcgggagc caacaaggcg ctgttgctta agatggccca agaagtggtt 6840
cacaccgcgc tggtggtatc cgagccgttc atggtcggct atggcttgga caagctgaac 6900
aataccatca ttgtcgatat cggcgccggg accacggata tctgcgccct aaagggcacc 6960

gtgcccggac ccgaggatca ggtgaccctg accaaggcgg gcaattatct ggacgagcgc 7020
ctgcagaacg ccattttgga acgccatccc gaattgcaaa tgaatacgaa tgtggcctgc 7080
gcggtgaaag agcaattttc attcgtcggc gcgcgcggcg aagcagccac cttcgagttc 7140
cgcgccgccg gcaagcccgt gcgttgcgat gtgaccgaat cggtaaagat cgcctgcgaa 7200
gcgctgatgc ccgatatcat tgaaagcatc gagattttgt tgcggtcctt ccagccggaa 7260
tatcaggcca ccgtgctgca aaatatcgta tttgccggcg gcggctcgcg gattcgcggt 7320
ttggcagcct atgtgaaaga taaactgcgc ccgttcggca atgcggacgt gacttgcgtc 7380
aaggacccga cttttgatgg ttgccggggc gctctgcggc tggcagaaga acttccgccc 7440
cagtattggt gtcagcttgg tgacgtttcc ggtcagtgat gggggatgtc attttggttg 7500
gcgaagatgc gtggctgtcc aggacattgg tatggtaaga gtgatcggat cgttggtgtt 7560
cggcggcttg atcctgctgc tggcatcgtc caacgcccat atggtggaaa cccgatttgg 7620
gccactgata atgcttgcgc cacatttcgt ggttcttggc attacgtttt ttcttggttt 7680
cgccattggg attgtgttgg tgtttgcgaa tgttatgaaa cggagcaagc ataaactgcc 7740
tgggaaaaac atcgtcatta agcgctgacg ttccatgctc cgtcggagct gggccggggc 7800
tttggagtag gtaaggatga ggaagagcgg ttgcgcggtc tgcagcagga gcatcggctg 7860
ggttggcctg gcggtgagta ccgtgctcat ggtgatgaag gcattcgtcg gcctgatcgg 7920
tggatcgcag gctatgctcg ccgatgccat gtattcgctg aaggacatgc tgaacgccct 7980
gatggtgatt attgggacca ccatttccag caagccgctg gatgccgagc atccctacgg 8040
ccatggcaag gtcgaattca tcctgtccat ggtggtcagc gtggtcttca tcgtgctgac 8100
cggctacctg ctggtccatg ccgtccagat cctgttggac gagagcctgc accggacgcc 8160
acacctgatc gtgctgtggg cggccctggt ttccatcggc gtcaatgtgg gcatgtattt 8220
ctattcccgc tgtgtcgcca ttgagactaa cagccccctc atcaagacca tggccaagca 8280
tcaccatggc gacgccaccg cctcgggcgc ggtcgcgctg ggtatcatcg gcgcccatta 8340
tctcaatatg ccctggattg atcccgcggt cgctctgtgg gagaccatcg acctgcttct 8400
gctgggtaag gtcgtcttca tggatgccta tcgtgggctg atggatcaca ccgcgggtga 8460
ggcggtgcag aaccggattg ttgaggcggc ggagcgcgtt cctggggtca ggggcgtcat 8520
ccatctgcgg gctcgctatg tgggccaaga catctgggcc gacatgatta ttggcgtcga 8580
tcccgagaac accgtggaac aggcgcatga aatttgcgag gcggtgcagg ccgctgtctg 8640
cggaaagata cgccgcatcg aatccctgca tgtcagcgca gaagcgcgcg aaatcggcga 8700
tacgaccaag ccgagtttct ccgatcagcc gctgagtttc gacgaggtca tgctgtccaa 8760
ggtggataac taggtggttg gatttatcac cctcgctgtg ttcatcgcga cgttcgccgt 8820
aatctatcgg tgggcggagg gcagccatct ggccgttttg gccggagcag cggtgttggt 8880
ggtgattgga accatcagcg gcacctacac cccgcgtatg gccgtgcagt cgatttattt 8940
cgagaccctt gcgttgatct tcggcatggc tgccatctcc gctcttttgg cccgatccgg 9000
cgtctatgcc tatttggcgg ccgggacggc ggaattgtcg cagggccaag ggcgctggat 9060
tctggtgatg atggctttgg tgacctacgg gatttctttg gccagtaaca gtcttatcac 9120
agtggccgta gtcgttccgg ttacgctgac cgtatgtttc cgcactggaa ttgatccggt 9180
acccgtgatc attgctgaga ttatcgccgc caatctgggc ggttcctcga ccatgatcgg 9240
cgattttccc aatatgatcc tggcctcggc aggcaagctt catttcaatg atttcatcgg 9300
tggaatgatg ccggcctgtc tgatcttgct ggcggtgact ttcttgtttt tcgagtaccg 9360
ccagggcgac tggaagaagg cggaaattcc cgtggattta gcttgggtta gaggcgagca 9420
gctacggtac agcgatatcg accatcggct cctgcgctat gggctgatca tttttttcat 9480
taccgtcatt gggttggttc tggcgggacc gctgaaggtt cggccgggct ggatcgcctt 9540
tgtcgccggt ctcactgcgc tggcgctggg gcgcttcaag gacgaggaat tcttttccgc 9600
ctgcggcggc agcgatatct tattctttgg cggattgttc gtgatggtgg gagcgttgac 9660
ctcggttggg attctcgatt gggccgtggc ttggcttgaa ggagttaccg ccgggcatga 9720
ccgcgtaaga gccattctgc tgatgtggat ggcggcgggc gtaaccattt tcgtcggcgg 9780
cggcacttcg gctgcggttt tcgccccggt ggcggcgacg cttcgcctgg acggcgatgg 9840
acaggcggct tggtgggcgc tggcccttgg catcatggcc ggctcgtgtg ccgcgctatc 9900
gggcgccacc gccggtgcgt tggccatgaa ccaatattcc ggcttcgtga agcggcaccc 9960
ggaattggct tcggctgccg ccgcgggatt gcaattcacc catcgggaat atgtccgttg 10020
gggattgccg ctgatgggga tttttttggt gttgtcgacc gtgtacatcg ccgttctcgc 10080
aggatgaccg atgacgacag gaactcgaat gaacgtggaa agcggccatg attgaaattg 10140
gcgagaccat gggtgatcag cccaccaaca aaatcgtctt ttgcgagcgg tcgtggaaag 10200
cgcctgtctc catcctggcg ttcctgatcc tagtgacttt cgcctggggg gcctatctcc 10260
tcgacaacta cgacgaggac gactacttcc gtggtagcga cgatatgtcg gtcggccaat 10320
tcctggtccg caacgtcgcc atgcctgatg tgcagcggct gtactataca gtaccaccgg 10380
cggtggtcgg tgtggggggc ggcggcgtca atgccggtcc cgtcgcttcg ggtgccatcg 10440
ttggcgccaa tggctatgtc attacgaccc tgcattccgt cgccaatgtg ccggacatca 10500
cggtccaggt tgccacctct gctggaatcc gtcggtttcc cgcccaggtg gtcaagacca 10560
ttccggccca taatctcgcg ctgctgaagt tgcagacgac cgagaagttc ttgcatttcc 10620
gcatggcaaa cattcaaacc gtggtccccg gccagcaggt tttcgccttc ggtcggaaca 10680
tggccggtgc gcctctggta cggcagggca tggtgcaatc ctccgatgct ccgctggcgg 10740

tggggaccac tcagatcacc catctgctgc gttccgacgc ggtctatagc tgggagcaga 10800
ccggcgggcc tctggtcaat gctcagggtg atctggtcgg tatcaacatt gccgcaaccg 10860
gtcccaccgg caaggtcgaa ggctttaccg ttcctgctca ggtgatcgtc tcccatctta 10920
aggacgtcgt gcgattcaag accggtggcg ccgcgggagt tgctcctccc gcggcccaaa 10980
ctgtggccat gggatcctct agctggtggt ctaaggccaa agcggtggtc ggagggccga 11040
ccgccgtacc gggaatgggc atgaatgtgg tgcaagggac ggtgacgacc gggattccct 11100
cgggcatgcc gtttgtcgat accgaccatg tgggaggggc caagatcggc ggatattcca 11160
tcgccgatat tctcggccta ggcatgcttg ccctggcggc cggtgtcacg ggtggcatga 11220
tgaccatggg cggcggagtg cttcaggtcg ctggcatgat ggtcttcttc ggctatggca 11280
tgtatctgat ccggccggtg gtcttcctga ccaatgtcgt ggtctatggc gcagcggcgc 11340
ttcgcaatga taaggcccaa ctggttcagt gggacaaggt gaaaccgctg atcccttggg 11400
gcgtcgctgg cgtggtcatc ggttatttca tcggtaacgc catcggagat tccgtggtgg 11460
gcgtgctgct tggtctcttc gccctgatca tggcaggcaa ggcggtgttg gaaattctgc 11520
aacccaatgc gggggaggat acggctgaag ccattgctgc cgccgaggct ggcgatgaga 11580
tggacgaact gatggctctg gcggaaggaa caaccaggcc caagaccagc ggcattgctt 11640
tgccagaggg gccgacccgt tcagcggtgc tggggctgcc catgggcctg ttcagcggca 11700
ttctcgggat cagcggcggc gtcatcgagg ttccgttgca gcgttatatt gggcggatca 11760
gtctgcagaa cgccattgcc aacagttcgg tcctggtgtt ctgggcctcg gtcgccggtt 11820
cagtggtagc ctttattcat ggcggcagca ccggtcttat tcattgggag gctccggtaa 11880
cattggcgct ggtgatgatc ccaggtgctt atgttggcgg cattctcggc gctcggttga 11940
tgcgggtgtt gcctgtccgg gtgcttaagg gtatctatgc ggcaaccatg gccgctatcg 12000
ccatcaagat gctgacaacg gtgtgatgga tatggttttg ggattagccg ggaatgaata 12060
gcaaactcgt cctgctggtg gtgggtgtcg tttttgcact ggtgcttgtc attgggcggc 12120
aggggggcgt cgttgctcct cagtcgatca gcgtttcgcc tcagatgtcg actgcggctc 12180
cggtcgctgc cccgattgct tttccgcagg cgaccaatgt ggccatggcg gtggagccca 12240
tggccgcggc gggtggtacc gtggctgcaa tggagtcgcc cttgcccaat ttcgttccga 12300
gcaatctcaa ggtctttgaa ggccattggc agggcatgga cggacgtctg atgaccgagg 12360
aactggcccg aaagttgaac tatcctcgtg gagtgcaggg cgttctgttg ggcgaggtta 12420
ctttgaacgc ggcgttcagc ggtctgttgg ggggggatgt cgtcgttcgg attgatgaca 12480
ctccagtcac caatatggaa aatttccagg cagctacccg caatgtggcg aaccggtccg 12540
aggcgcggat cagcgtgatc cgtaaggaca accgtcccgg caccccggtc ttgcgcaaac 12600
tgaccgtggt tttgcgcgcg gccgaaggcg gtttgggctt tgcccagttg gaaggcgccc 12660
ccatgatcct tccgggcgat cctcggcccc atggatatcg cggcgcctgc accgattgcc 12720
atccggtagg ccagggattc gaattgtcgc ccgatcctga tctcatcagc ctgccgcctt 12780
cggccataac gcgtgatgcg gttacccggg gcgtcagtcc ccacgaagtt cgtggcccgt 12840
gcgaagcttg ccacgtgata aattagccag agccaccgga ttgggatttt ggagaacagt 12900
atgtctagca agccgtcgaa tatgcttgat gaagttaccc tgtatactca ctacggccta 12960
tccgtggcga agaagctcgg tgcgaatatg gtcgatgctt tccggtcggc cttttcggtc 13020
aatgatgaca ttcgtcaggt gtattaccgc gacaagggca tctctcacgc caaagccggt 13080
cgttattccg aggcagtggt gatgctcgag caggtttacg atgccgatgc cttcgacgtg 13140
gaagtggcgc tgcatttggg aatcgcctac gttaagaccg gggccgttga tcgcggcacc 13200
gagttgcttg agcgctccat cgccgatgcg cctgacaaca tcaaggtcgc gaccgtcctt 13260
ggcctgacct atgtgcaggt gcaaaaatat gatttggccg tgcctctact ggtcaaggtg 13320
gcggaagcca atccggtgaa tttcaatgtc cgcttccgtc tcggggtggc gctggataat 13380
ctggcccgct ttgacgaagc catcgacagt ttcaagatcg ctttggggct gcgtcccaat 13440
gaaggcaagg tgcatcgcgc aatcgcgtac agctacgagc agatgggctc gcacgaagag 13500
gctttgccgc attttaagaa ggccaatgaa ctcgatgaac gttcggccgt ctaagcagtc 13560
gatgtagaag gtctcattga gatgacgata gcaacgaggc ggatatggca gtaagcgatg 13620
cggacgccag ttcggtcgat aaggtcgaat ccattaccct acaacgggtc aagcagtcgg 13680
aggaactgct ggcccaattg tatgtggttg aggaatcgcc ccggcgcatg ggacgggggc 13740
cggtgcaact catgctggcc atctcggtgc tgtcgctggt tgccttcatc accaccttgc 13800
tgatgcgcta taacgccttt gtcaccatgt acgaggatgc ccaggccaag cgctccaatt 13860
tcgaggtcat gattcaacgc cgcgacaatc tgtttggcaa tttggtcaag ttgacgctga 13920
atcatgccgc tttggaacac tccatcttct ctcacacttc cgacaagcgt aaggagtccg 13980
tggaagccgg caagggcggc ccgatcggtt cggccatcga gcaattgatg aagcagggcg 14040
gaatcggaaa attgctgggc gatggcgggg ccggcaaggc cctgttgggg gccgatggtg 14100
gtttcggcaa cgcgctgggg cggctgatgg ccattgtcga gcagtatccc accgttcagt 14160
cggcggatac ctacaagcat atgatgacct cgctggtgga tatggaagac cggatcgcca 14220
gcaagaggga ggaatttaac gcatctgccg cgacctataa tgtggcaatc accaagtggc 14280
cgtgggatta tctggcaatg atcaccgggt tcaagcgcgt ggagtatttc cacgaaaagc 14340
ctgcaggcga cacgccgatc attacgccac agattttcca ggaactactg cctctcaccc 14400
attcgcagga atccaagaat tgatttggac agcagtgatc aagggaagtg ccttgatgac 14460
ctttgttcag ggcgccatgg ctctggtgga caaggtattt ggcgaggaaa ttctgccgca 14520

ccgcatctat agcagcggtg aagcggcgca gttgctggga atggaacggc tgcaggtgct 14580
tgagatggtc cgggcgggga cgatcaaggc gaagaaggtt ggcgataatt atcgaatcct 14640
gggctccaat cttgtggaat acatgaaccg atgaagttcg aaaattgcag agactgccgg 14700
gaagaggtcg tgtggtgggc attcaccgcc gatatctgca tgaccctttt caaaggcatc 14760
ttggggctga tgagcggcag cgtcgttctg gtggccgatt cgctgcattg gggtgccgat 14820
gtggttgcca gcggcgtgac ccagctgagt cttaagatct cgaataagcc tgcggatgag 14880
cgctacccgt tcggatacgg aaatatccaa tacatctcgt cggccatcgt ggggtcgctg 14940
ctgctgatag gcgccagctt cctgatgtat gggtcggtgg tgaagctgat ttcgggcaca 15000
tacgaggcgc ccagcatttt cgccgccttg ggcgcgtcgg tgacggtgat cgtcaacgag 15060
ctgatgtatc gctaccagat ctgtgttggc aacgaaaata acagcccggc catcatcgcc 15120
aatgcctggg acaaccgttc ggacgccatt tcctcggcgg cggtgatggt tggagtgatc 15180
gcctcggtga tcggttttcc catcgccgac accatcgccg ccatcggcgt gtcggccctg 15240
gtcggtgaca tcggtctgga attgatcgga aaggcggttc acggcctgat ggacagctcc 15300
gtggataccg aactcttgca gacggcttgg cagatcgcca cggatacgcc gctggtccac 15360
agcatctatt ttctgcgtgg acgccatgtg ggcgaggacg tgcagttcga tatccgtctg 15420
cgcgtcgatc ccaatttgcg gatcaaggac agctcgatgg tggccgaggc ggtacgccag 15480
cgcatccagg atgaaatccc acacgcccga gatatccgcc tgttcgtcag cccggctccc 15540
gccgcagtga cggtccgggt ctgatcagga gaggggaatc atggactttc ggcctgatca 15600
agtggtagcc cgcatccggg gcgcggtgga aggcgctttg accgctcaat ccgtgcttgg 15660
aatcgggggc gctctagtct tgattctggt tgttatagcc ctgcttcccg atcgctttac 15720
ccgtggggaa ggcaagaccg ccaccgccgt gtcctctggc gccgcgcagg cccttcccgc 15780
cgccttgcct gggttatccc ccttcacgcc agccaagccc ctgcagttca gcgggcgcgt 15840
cacccaggtt gccagcatcg gcaatgatgt gggctggggg caggtccatg tctggatcga 15840
caacggtaca ggggccttgc aggaaatttc ggtggcgccg caatcctatc tcaaccagat 15960
tggctgcccg tcctttgacg gggcgcggat cagcggcatt ggtttcttgt tcgatgcggg 16020
acgtcccaat gccgagcttt acgccaagtc cgttctggtg ggggggcgga catgcaagtt 16080
gcgcgatgac gagggtttgg cgctgtggat gaccgtgcag tgagccatat gggacagaga 16140
gggtgaacat gggtacgcca gggggcggcc gtcgctggat gaccttgatc tcgatcacct 16200
tgctgatggt ggtcggactg ggactctatt gggatgagct gtccctctcc gccggcatct 16260
cccccgccac atcgccccgt cgggcggagg ggcttttgtt ggggcggctg cccttgccca 16320
tggagccttc gattctgtcg ccgctggagc atctcattga gccgccgctt cagtacaagc 16380
tgatgaccat tcgtcatatc ccgccggtaa tgccggggac aggcatgccc catccctatg 16440
tgggggattg catccaatgc catctgatgg tcggtggccc cgctgccgga tcacagttca 16500
agacgcccta tggcgccgta ctggaaaacc tgtcgcgggt ccgcaaactg gggcctccca 16560
ttcttcccac gacgcgccag ccgcatccgc ctgccgggcg ctgcattaag tgccatgaca 16620
ttgtggtcaa ggtgcctgtg gaaaagaagt ccggcattaa atggctgttg taagcgccgg 16680
tgtcgtcctt cgctgaacag gggctgtgtg gaagatgcgg atcgccgcaa tcatcaatgc 16740
gcgggccgga accgtgcttc ggatgtcgcc ctccgccgtg acggagcgac tatcggtcgt 16800
ttggggctcg cttgggcatg atgccgcgat cattcttgcc gaaggtaagg acatggggcg 16860
gatggtccgg aaggcttgcc gcgatcccga catacgggcg atcatcatcg gcggcggtga 16920
cggatctctg tcacgggctc tcgaacatgt gctcgcaagc ggcaaatcgc tgggtgtcct 16980
gcccctgggc accatgaact acatggcgcg tcagatcgga atgccgttgg atctggccca 17040
ggcggcggtg gccttggcgg gagccgtcca gactcccatg gatgtgggac gggtcaatga 17100
tcgttatttc cttatccgag cctgtttcgg gcgctttccc gaattcattc agtcgcgcga 17160
ccgggtccgg cgcaaggggg gaagctttct tgaaggcgcc ctggccggat tgagcggagt 17220
ggcgcggcgc tatcggattg tggaggcgga attgtccagc ccaggggggc gcgcccggat 17280
cgccacaagc ttcctgatga tttccaacaa tctgtgccgg gactccgacc cgttcctgct 17340
ggagcgtgaa cggatggatg gcggatccct ggggggttat gtggggcgca gcgccggacc 17400
ggtcgggctc atggaattgg gacttcaggc cgctatgggg cggtgggcca gcaacgaagc 17460
cttgttccag ggtgagatgc actggctgga ggttcaaacc gaacagcgca agccgctgat 17520
ttccattgat ggcgaggtgg agaaaatgga aggtcccttc cgcttcgaca ttctgcccgg 17580
agcgctttcc atactggttc cgaaataacg ccgccatggc catggtggtg cagttgcctt 17640
ccataccgcc ctactgccat ggaactggcg cccgctgccg cgcgtcgccg acagtcaggc 17700
agcctgatcg gtcagcaggg tggccccgga gccgactagg ccggcgtggg taggcttcct 17760
cgagatctta tccctgcatg gttatttcat tcaaaggggg gagtgtcaga ttacagtgcg 17820
ttgactgata cttttggcgc gaccgaatct acgcttgcgc gttgcgccgt ttttaatccc 17880
gtgttaccaa tgaattggaa acttttgttc acgttgcccc gaagagatgg gctcggtcat 17940
gaaaaatatt gcggaccccc ttccgtttga cggtgacgaa ccaatgccca ggcgccttca 18000
tatcgtcagc gggcaggtgg aaccgttgcc ggacgacaac acggaccagg cgttgatagc 18060
ccaacagccc gcagccatgg aggagacgga catcgtcgct ctgctgcagg aatgtgaggg 18120
cgacgaggac ggccttccca ccgcggcctt cgataccccc ttcccttccc ttcccaaacc 18180
cgaacccgaa gcgctggccg agaaggtacg ggcccctgtc cctatgtcag gcatcggcga 18240
tcacggggat gccatcatgg ccgtgattcg tgacgccctt gaggcggagc ggcaccatgc 18300

cgatcaggac cttgtggcgg ctcgcattcg gaccgaaacg ctgagcgccg agatcgacga 18360
cctgcgccgc tcggtcgagg atgttcgccg ctcggcctca gagcaggtcg aggccactcg 18420
cgcggtggcg gctgagcaga tcgaggccgc ccggtcggat gcggccgagg ccattgggca 18480
actgaccctg acgttggatc aagttcggca gttgcgggct caggtagact cccagagtaa 18540
acatttatcc gccctgatcg gcattgagga aaaagcccgt agcctcgacg cgcaattggt 18600
cgaagagagg acccgcaacg ccgaattaca ggagcgcatg caggtggtcg ccgagggccg 18660
gactgctgcg gaggaaacgg ccgcgaaggc gcgggcggcg gccgagttgg cccgacgcga 18720
actggccgcc ggaagggcgc tccatgccga accgagtggt gtcggcgaga ttgtggcaga 18780
acatcgtgtt ccggtggtgt atggcggcgc cgtgtcgggc gcgctgtttc cggttcagat 18840
taccgcggtg gtcgaaaccc agacctcggc cgaagatcgc accggcctcg gcgccctatt 18900
cgatatccgg ctcgccaccg ccattccttt ggacaccgcc agtgggccgg catctcccca 18960
cgaagtggtg gaccgggtgg tcagcctcgc ccattccatg tatccgaacg cggtgctgac 19020
catcgctttc ggcgacgatg gcgaccgccg ggccgtacag ggccgctatc aggatgtcac 19080
cttcacctcc gccgatgtcg atgaccgtcg gcgcaaggcg gcccaagtgc tggccatcgc 19140
cgcaggcaac ttgtcgcagt agcctgatcc gctatggtaa gcgcatcatg tccggatccc 19200
atggcgttcc gctcgtcctg gtgtccctgt tgataccggg aagccctggg ccaacttttg 19260
gcgaaaatga gacgttgatc ggcacgtaag aggttccaac tttcaccata atgaaataag 19320
atcactaccg ggcgtatttt ttgagttatc gagattttca ggagctaagg aggctaaaat 19380
ggagaaaaaa atcactggat ataccaccgt tgatatatcc caatggcacc gtaaagaaca 19440
ttttgaggca tttcagtcag ttgctcaatg tacctataac cagaccgttc agctggatat 19500
tacggccttt ttaaagaccg taaagaaaaa taagcacaag ttttatccgc cctttattca 19560
cattcttgcc cgcctgatga atgctcatcc ggaattccgt atggcaatga aagacggtga 19620
gctggtgata tgggatagtg ttcacccttg ttacaccgtt ttccatgagc aaactgaaac 19680
gttttcatcg ctctggagtg aataccacga cgatttccgg cagtttctac acatatattc 19740
gcaagatgtg gcgtgttacg gtgaaaacct ggcctatttc cctaaagggt ttattgagaa 19800
tatgtttttc gtctcagcca atccctgggt gagtttcacc agttttgatt taaacgtggc 19860
caatatggac aacttcttcg cccccgtttt caccatgggc aaatattata cgcaaggcga 19920
caaggtgctg atgccgctgg cgattcaggt tcatcatgcc gtttgtgatg gcttccatgc 19980
cggcagaatg cttaatgaat tacaacagta ctgcgatgag tggcagggcg gggcgtaagc 20040
ttacaatttc cattcgccat tcaggctgcg caactgttgg gaagggcgat cggtgcgggc 20100
ctcttcgcta ttacgccagc tggcgaaagg gggatgtgct gcaaggcgat taagttgggt 20160
aacgccaggg ttttcccagt cacgacgttg taaaacgacg gccagtgagc cggcgtaata 20220
cgactcacta tagggcgaat tggagctcca ccgcggtggc ggccgctcta gaactagtgg 20280
atccttcatg tactgcggaa cagtcgcggc gtctccatgc tcgacgcagt tgtggcgggt 20340
ggcagcaacg cctcctcggc cttaaccgac cgcgtccact caatggtggc gacaccagcg 20400
caggacgggc aacaagccgc ccacgcctcc accacgcgag cacaagcacc acagacccaa 20460
gcaggagaga gcgaagcctc atgctcccgc cgccgccaga tctcagccga ccgcgccgac 20520
cctccaggct ccctttcctc gatttcggcc atcagccgac atcccagcgc atcgggagcg 20580
atcttcaatg ccgccatgat gtggcggcgt gccacccccc atttctgaac ggcaaaggca 20640
gcctctccgg cggccaagtg cccatcggga tgatgggggg cgattgccgc cagggcctcc 20700
agcctcttca ggcaagcatc ctgatcctcg tcgcgccaca atggtgcaca agcatcaagc 20760
agcactcgag caggaacatg tggccaaacg gacgccagca gggtctcggc ctctgccttg 20820
cgtccctcgg aaacctcggc ccggatcacg tgcaacgcag gaggcagaaa agtctggtcc 20880
gccgccatag cctcgcgcgc cagacgcaag ggacgagcgg gatcaccggg ggatgcctcc 20940
aaagcttgtc cctcaagcgc caccgatcgc caccgcgcag ccaccaaggg cgtcagccga 21000
ccagccagaa ccgcctcacc aatccaggcc tgggctgccc cccattcccc agcccggatc 21060
ttggcggaaa aggcctctaa atcctctcgg ctcggcccgg cgggagcggc ggcgagtttt 21120
tcaacgacga caggctgtat cttatccacc accggagtcg gcggtggtgg cgggcccggc 21180
tttcgcgcga acagacgccc cttcgcggcc tccaaccggg tcggcgccac cggctgtgcg 21240
ggcgggcgcg gcaaaagccg gtccaggcgg gcggcaagtc cgggattgtt cagcagccgc 21300
gcagcctcgg ccgcgaagcg gcggccttcg ccgacatccc cactctctgc cgcatccagg 21360
gccgccagca aggcactcat gcctttttcc agcccccgtg cctgccggga tttccccagt 21420
ttggagggta ggtcggccac caatgccgaa agacgcgaca gggcggaaaa aatcagaaac 21480
accaccagga tcacggccag aagaaccggc atattgctgt ccacgtgcca gccaagccat 21540
tccacctgca ccgatccgac attgtctgag aaccacaggg tggcaaacac aaccggcgac 21600
atgaaaatca acaggacgat caggcgtaat agcatggatc actcgtctcg agacgaggcg 21660
acggtcatgg ccatcgccga caattcagac aatgcagcat ccgcggacag acgcaattcc 21720
gccgcctcca gccagggttg gatggccgca agggacggcc cttccgcgcg acggagcaag 21780
gcggcagccc cggctaaatc gcccccggcc agcaaccggg aagtgctgtt caggatacca 21840
tccagcccct cttcgccccc ctcggtgcgt ctgatcaata ccgcacctcc cagccaccgc 21900
cggatatttg gtggcaccca accggaatcc gcggggacat caagcttacg tgccgcggcg 21960
gccgtcaccc gaaaactttc ggccaaaccc acacgggtga cgattcccgt cgccgagccc 22020
atcaccagcg gcgccagctt atcggcggtg cctttctcgg ccagtatcat ggccgctttc 22080

atctgtgcag ggtaaggact gccccggtca accgcttccc gcaattgtcc gagtgcggcc 22140
aggaacaagg gcgcgcgatc ccgccgctcg gcgatacgtc ggaaagccgc ctccgccttc 22200
tccaccgcac cggagagctt ggttgcgccc ccttcatccg ccgccaactt gcgaacaatc 22260
tccaagtccc ctttaagcgt tgcgatctcc gccgccatcc aagacatctg ctccggatcg 22320
gcggaaacac cagccgcctc tttacgaggg tcttcccccg ccgtgcgggg attagcctct 22380
agcatggcta ggcgccggtc ggcctccgcg atccgcatag ccaggggagc caggcggcct 22440
tccaccatat ccatccgttc atccagggtc gcgggacgcg gagcgccaac agatccgccg 22500
tcacctatcc aggacagcaa tgagccagga aaactcccaa ggcgaggtgc caaccaaggc 22560
catgccagca cggctacgac cacggcgatg gccagataca gaaccaggac ctgtccacca 22620
ccatcccggc gtcgagccgg acggcgacca ctcggtcccg gtgccttttc gttgatgtcc 22680
attggctccg cttccgctag ctgcgcggcc tgcaaccggg cccaaccgcc ccgcaacggc 22740
gattttcacg atcaccgatc cctttggcaa gacattaata ttatagatga aacggtcctg 22800
aaacagctga tgccctgcac agccgggcat cagacgtaaa tcacccgagg ccgaacctca 22860
gatccggtcg gccacccagc tgaccacggg cagtttccat gcccgctgga acaccacgga 22920
gaccaagccc agaagggaga ataccaggac ccccatggat gaaaacccga aaatccactt 22980
ccccagaacg ggaacatgca gcgcgaacag cgccagcacg ccccacatcc agatcaccag 23040
accctgcttg gcgtggaagt agacaaactc gtcgtcccga tccaccagca gcggaacgaa 23100
gcacaggacg ctgagataag acagcgccgc catgacggtc gtgcgcgcgc caagagtgct 23160
ccgaaggatt gcttcaacca tgtccccccc cccccgttca ggacagcgcg tcgcgcagtt 23220
cgacttcttc atcgctctgt gccgcttcga tatcacggct cttcatatag gcgtagactg 23280
ccccggcccc gacgacaccg aggatgagcg gcccccaagc cccaaggccc ataccgagcc 23340
ccagggcaag ccccttaccg gaccagatgg tgccgccgac agccttggcg ccggcgccga 23400
ccttcccaac gccaaccgca gcggccttgg ccccagcgcc ctgcgccgcg acaaccttgg 23460
ttccagcccc ctgcgccgcg gcgggcgcgg ctccggcctt ggcagcgcca accttgcccg 23520
cgccaacctt ggcagtcgcg ccttcgcgtt ccatttcacc caggcggcgg gccttggttc 23580
cgaccggaac cccagggggg caaaccattc cgttggcgat ctgagcaggc accagagttc 23640
tccttagcaa tcaagtagtg cgggactgaa aaacatcagc ctcaccagac atacaattca 23700
ataccatgag gttgccgaat aggacaacct gtatggggct aaaaaacact attacatgga 23760
agttacgagc gattattatc tgcaccgccc ccgctagctg cgggttattc gcatttgcct 23820
ctgcgcgggt tttgacagca gcccctgttg cacccggccc cgctgtcgcg gttcttgtgg 23880
acacagcgaa gcaccgcagc tcccgccaaa accacaagcc caataattgt cgacaaatcc 23940
caaagagtaa gcacgggacg atcccttaac gccaatgacc accaccacct taaacctccc 24000
cacagaaatc tgccaccgtg agcaaagcac cctgtttggc aggcatggcg cgcagcgatg 24060
accagggaag gaattgacag gtggagtcca gtcagcccta cctaagcgcc cacgacagca 24120
taaggaataa ctctattttt gcacaccccg tcgcctgaag ccaccttgac agaaattgat 24180
atctattctc aactttttcg ctttactagc tcttagttct ccaataaatt ccctgcgtcg 24240
attttaaggg gcagagaggg aatcgtgcat acacttgccg gagatcagat gatcaagggc 24300
atcgcgggag ttggcggaac cgcccttggc gtcggtggcg gagttgccgc ccctccggtc 24360
tctgccgctg ctgtcggcag caccttgctg gccggcaagg gggtgtgcct ggggctgggg 24420
cttggcctcg gtgcttgggg tcccgttctt ctcggcgttg ccggattggc ttgcgccgcc 24480
tccctatgtg attatctgaa gaatcgcaaa gcgcaggctg aggcctccgc cgagcctgct 24540
taagcgaggg caaagcaatg gccgagacta ttttgatcga aactaaaacc gctggcggca 24600
actgccgttc atatctgatg gcgggcgcta gctatctggg catcctctgc ttcgtcccgc 24660
tgcttatgag ccgcgatgac gaatatgtgt acttccatgc caagcagggg ctggtgctgt 24720
ggatgtggag catcctggcc atgttcgcgc tgcatctgcc gggcatcggc aagtggctct 24780
tcggcttctc gtccatgggc gtcctgatgc tgtccgtggt cggcttggtc tcggtggcgc 24840
tgcgccgcac ctggcgtctg cccctgatca gccatgtagt cgccctgatc tgacggcgag 24900
cgatctaacg gacccggaca gcagcgcaag caggcgaccg gagtcctttg ctttggagtc 24960
ccaagcggct tctgccatgt aggcgcagtt tcgtctcagg aaaggccaat accatgcagg 25020
acctttttct cgccaaggtc gaaagcgcca tgcaggcgtc ccaggtcggg gcacttgccg 25080
gtcagacggc gacggtctcg tcagtctcgg ccacgaccaa tctggccacc ataaccccaa 25140
ccaccgccgg gcaggcccct atcatcgtca aactggacgc ggcacggcag gtgacggagt 25200
tgcaggccct gatgggaaag accgtgctgg tcggaaagac cccgaccacc atcggcggca 25260
tcggaaactg gattgccttg accccggcgg cgggagccaa gaccggcgct gccgtggccg 25320
gaaccggtca gctggtcatg atgaaggtcg agggcaccgg cgcggccatc aagcttcccg 25380
ccctggcggg taagagcttc atcgtcgccc agccccccgt agccgccgga accaaagcgg 25440
cgggcatgct ctatctgaat ccggttggcg gtggtgatat ggtggccatc aacattcaga 25500
acgccatgac ccagaccggc ggcttggtcg gcaagacctt caccgtcgcc cccagccccg 25560
tcattggcgg caccaccggt aaattcctgg tcctgaagcc catggcgacc ggggtcggca 25620
aggcggtggg cagcggcgcc gtcgtcgcca agttcgtacc cgccgccgtc accggcacgg 25680
gcggagcggc ggctatcggg gccggatccg ccaccaccct gatggccacg ggcgccagta 25740
cgatcacccc cgtcactgcc gccgccgctg gcagcgccat gctgacagcc aaaggtgttg 25800
gcctcgggct tggcctgggc ctcggcgcct gggggccgtt cgccctaggg gctatcggcc 25860

tagcgggtgt tgtcgcgctt tatacctggg cgcgccgccg ccatggcgct cccgatgttt 25920
ccgatgacgc tcttctggcg gctgtcggcg aggaataagc ctgacccttg aattaaggac 25980
aacagcgatg agctttcaac ttgcgccgta cttggcgaaa tccgtccctg gaatcggcat 26040
tctcggcggc attgtcggtg gcgccgccgc ccttgccaag aatgcccgcc ttttgaagga 26100
caagcagata accggcacag aagcggccat cgacaccggc aaggaagccg ccggcgccgg 26160
gcttgccacc gctttctccg ccgtcgccgc caccgccgtc ggcggtgggt tggtggtctc 26220
gttgggaacc gccctaatcg ccggcgtcgc cgccaaatac gcctgggacc tgggtgtcga 26280
tttcatcgag aaggaattgc gtcacggcaa gtccgccgag gcgacagcgt ccgacgaaga 26340
cattctgagg gaagaattgg cctgaaatat tgggctggtt cacggcattc agacaccggc 26400
ggaggccagg gcgttggttg ttgttatcta aacaacgccc tggcagaacc gaacaagaac 26460
actgtcgtca ttcaccccga tgtgctctgg cgccgagcgt aagcatccgg tgagaacctc 26520
attccctcat gatacagaga ccggggactt atcagccaac ctgt 26564

<210> 36
<211> 5549
<212> DNA
<213> artificial sequence

<220>
<223> gene expression cassette comprising the mamXY operon of
Magnetosprillum gryphiswaldense

<400> 36

acaggttggc tgataagtcc ccggtcttca cgctgccgca agcactcagg gcgcaagggc 60
tgctaaagga agcggaacac gtagaaagcc agtccgcaga aacggtgctg accccggatg 120
aatgtcagct actgggctat ctggacaagg gaaaacgcaa gcgcaaagag aaagcaggta 180
gcttgcagtg ggcttacatg gcgatagcta gactgggcgg ttttatggac agcaagcgaa 240
ccggaattgc cagctggggc gccctctggt aaggttggga agccctgcaa cgtataatat 300
ttgcccatgg acgcacaccg tggaaacgga tgaaggcacg aacccagttg acataagcct 360
gttcggttcg taaactgtaa tgcaagtagc gtatgcgctc acgcaactgg tccagaacct 420
tgaccgaacg cagcggtggt aacggcgcag tggcggtttt catggcttgt tatgactgtt 480
tttttgtaca gtctatgcct cgggcatcca agcagcaagc gcgttacgcc gtgggtcgat 540
gtttgatgtt atggagcagc aacgatgtta gcgagcagca acgatgttac gcagcagggc 600
agtcgcccta aaacaaagtt aggtggctca agtatgggca tcattcgcac atgtaggctc 660
ggccctgacc aggtcaaatc catgcgggct gctcttgatc ttttcggtcg tgagttcgga 720
gacgtagcca cctactccca acatcagccg gactccgatt acctcgggaa cttgctccgt 780
agtaagacat tcatcgcgct tgctgccttc gaccaagaag cggttgttgg cgctctcgcg 840
gcttacgttc tgcccaggtt tgagcagccg cgtagtgaga tctatatcta tgatctcgca 900
gtctccggcg agcaccggag gcagggcatt gccaccgcgc tcatcaatct cctcaagcat 960
gaggccaacg cgcttggtgc ttatgtgatc tacgtgcaag cagattacgg tgacgatccc 1020
gcagtggctc tctatacaaa gttgggcata cgggaagaag tgatgcactt tgatatcgac 1080
ccaagtaccg ccacctaaca attcgttcaa gccgagaggc agcctcattt aaacattcag 1140
gacgcgctgc catattatac tattccgaaa ttaatactta gcaccacttt cccaacggac 1200
tcttaggcaa tgcaggtcct cacaaacaaa aggcggtgtt gcgattaatg cgcatcttcc 1260
ggtattgacg gactcgccac aaagccatat ctatcccttg tgtccaagct accttgccgg 1320
tcagcttgct gttactgtgg cagtatccta tacacaccag aacattttgg cgaccaccac 1380
agccaccacg ggagcagccc ttatgttgat gaactttgtc aacaatgtat caaagacgat 1440
taacggagga gcccgcatcg tatatgtcgg atcgttctcc tgggctgtct tatcacttct 1500
ttttgtcacc gctttcagtg gctggaataa tattttctca atgctccccc acgagatttt 1560
tattctagtt ctaaccattt cacttccgat tgctctgatt gttttgatct tcatgctctc 1620
gcagattgtg agaaccgtcg agagcgtgaa gtctgaaatt tccaccctgt cccagcggga 1680
tcctgtttcc gaagaggcgg tcaccatgct ggccgatctg ttccgcgaac atcgcgatgc 1740
cgttgccgcc caggtggcag cccaagtcga ggccaccgcc cagttggtcc agatcaatca 1800
ggacaaccgc gccctcgccg cgccctcgcc ggattcaggc gacgaaaacc ccctggcctt 1860
gctggcccaa atgttccgcg aataccgcga gacagtgacg gcgcagctgg aagcgcaaat 1920
ctcggccacc acccagttgg tggaggcaag ccgtgacagc cgcgacggca tcgtcgatga 1980
actgcgctcc caacgggttc tgtcccagga aatcacccaa gaactatccc atatcgccca 2040
aagccggaat gtggttccgg tcgccgagcc cggcctcgac ccgtcgcagc ggatcgaccg 2100
catgcgggcg ttggcggagg tgctggggct ggccctcaat gacctcagca tgaccgcaac 2160
ccagctcctg agcgagcacc tgaacgcggc ccatggcgac agggagggaa cacaaaagtt 2220
tatttcgacc ctgactaacg cctatttcgc gggtgacaag aacgtctttt tccgcagctt 2280
ggtcagcgag gtcgtcaacc attccgacca attgcagcaa tgcgcgattg gtgccgagaa 2340

tgtgcgccag cagatttcca aaatcctgcg cgaggcccgt gagatccgct cgctggtgtc 2400
cgcctgcgac cctaatgatc tggtccggat cgtcttcgag gatggcgagt tgtgggcctt 2460
ggaaaaggcc ctggccgagc acttcctcat cgatggaacc cccatctccg atgcgtgacg 2520
aaagccgacg tccgaaattg catggccgcc atttccagct acggtccaga cggaaggccg 2580
atggtcattc cattgcgtat gggaatatga gatacttcgg cactttcatc gctagcgcat 2640
cgggaatcgg tagctccact ttgaccacag tcgaactccc gtgaacacca aagccgttgc 2700
acatcccgac atcgccgtct ggatcatggc gttgggcatt gccttcagca tggccctagt 2760
tctgaccgcg ctgttcaatg ccaatccgtg ggaagatcac acctacgatc tggcgcctcc 2820
cattgtcgct gggatggccg ccccccatcg cgatggccgc gagaagatgg tctgctccag 2880
ctgccatatc gtcacccccg cttccgccgc taccggtccg ggcgcgggca cgctgcccat 2940
cgttgaggga accccggcgc cgcatgtgga tggacgcgag aagatggcct gcgccagttg 3000
ccacaccatc gtgaagaagg gcagcgttgc caagagcggc aaggcaagcc ccgctccggt 3060
ggcattctcg cagggcatgc ccttacccga ggcgatgagc gtggcgctgg cggttacccc 3120
ggcaccggcg cccctgggca acgaagcgca tgagcggatg gtgcccttcc gctatcaagg 3180
caagatcgtc agcgtggcgg gcgctggcac ccgctcggtc tggggtgata tctatattca 3240
gatcaacgat gggatcaatc ctcccatgtg gatcgacctt gccccgctct ggttcctgca 3300
agcggagggc tgcttggtcc gtcccggtat gttcgtcaag ggcaccgcct tccgcgaccc 3360
Gacccaggcg agcgccgggc tggattacgc catgagcgtc atggccaatg gcgaagtctg 3420
tgccctgcgt gacgaccatc tcaacggcct gtgggctaat gttggaggcg tggatgccga 3480
agagcggtaa accctccacc ggcacgacac cggctgactt cgctcccacc caatggaata 3540
tcatctatct gctgatgacc gtgggctctc tggtggccgc gctgtccatc tctatccagc 3600
ccctgctgct ggataagatt ttcggcattg ccttcgaaaa agagggcgcg gtcaacgccg 3660
atattcaggt ggtggcagag atcgtctcca tcgtctgcgt gggatggttc ggcctgctat 3720
ccgatcggat tggccgggtt cggatcatcg ccacggcttc cctgatcgcg gtggctggcg 3780
cggccatgtc cctgctcagc ctgcaaattg ggctcgcctt cggtgccgcc gggctggtgc 3840
tgttctatct gacccgggtg ctgctgacag tgggggccga taccgtccag ttgcagcttt 3900
ccaccctggt gggcgatgtc tcctcgcggg ccaaccggcc gcgcctgatg ggaaatttgg 3960
tgttcatgat ggtcttcggc ggcaccatgc tgtcggccat catcatgcag atggccgatt 4020
ataagggcgg tgtgttcatc atcatgtgcc tgccgctgct gatcggcatc gccgggttcc 4080
agatgacgcg cgaatccttg cgggatgtag cacagcccca gcaagcgccc acgggagacg 4140
agcatcccct tcggcaagtc tggtcggtga tcaccagcga cccgcgcttg caattggcct 4200
tcgccgccgc cttctacacc cgcgccgacg tgatcatcct cagccttttc ttctcgctgt 4260
ggtgcatttc cgtgtcggat ctggtggggg tgacccgcac ctacgccacc gcccatgccg 4320
ccgtgatgat cggtctattg gggctggcgg ttctggcggc aatcccgctg tggcgatcct 4380
tcatcgagcg ccacagccgc atttccgcca tcggggccag cctttccctg gcggcggtcg 4440
gctatatctg gctcggaatg ttcgccaatc ccttcaactg gctggtggcc ttgccgttgc 4500
tgatggtagg catcggccat gccggatgtt tcgtgactct tcaggtcctg actgtggacg 4560
tctcgcccaa gccgatcctg ggcgccatgg ttggggcggg ctatctggtg ggcggccttg 4620
gcaccgtcat gctggtgcaa agtggcggct attatttcga tgcgcttggc ccgcgcgcgc 4680
cattcatcct gatgggaacc ggcaagatgc tggtgaccct ctacgccgcc tggctgctgg 4740
ccaacggcat tgatgagacc tgcgatcacc accttaaatc cacccgcaag gtggattgga 4800
agccgctggt gttcctgacc gcggccctcc ccttcgtctg gctgatcggg cgcagcgtta 4860
tcgaaggata tttctccaac ggctctctgg gcgaggcccc ggtcggtttc gtcaaccgct 4920
atttgggcga ttgggccttc accttcttga tcatctcgct gtccatgcgc ccggtgcagg 4980
agattactgg catcaagtcc ctggccaagt accggcgcat gatcggcctg ttcgcctttt 5040
tctacgcggt attgcacgtt ctggcctatg tcaccctgga atgggcgctt aatctgggcg 5100
acatggcgag cgacatctac aagcggccat tcattctgct cggcctggcg gccttcctcc 5160
tgctgatccc cctggccttc acctccacca acagccagat caagaagatc ggcggcaaac 5220
gctggaaaag gctgcatagg gccacctatg tcatcaatgc cctggtggcg ctgcatttca 5280
tccttgctgc caaccacgag aatggcgaac cctatgtcta tgcggcggcc gtcatagttc 5340
ttctgtggta ccgtttctac cagtggcggg gcggcaatgt gctgcgcgcc ctgcgaatcg 5400
gctaaagaat tcgatatcaa gcttatcgat accgtcgagg tcgagggggg gcccggtacc 5460
cagcttttgt tccctttagt gagggttaat tgcgcgcttg gcctcattcc ctcatgatac 5520
agagaccggg gacttatcag ccaacctgt 5549

<210> 37
<211> 4163
<212> DNA
<213> artificial sequence

<220>
<223> gene expression cassette comprising the feoAB1 operon of
Magnetosprillum gryphiswaldense

<400>   37

ctgtctctta tacacatctg acgtcgagcc acggcggccg aagcaggggg gcaaggctga   60
aaagccggcc cccgctgcgg ccccgaccgg cttcaccttc aacccaacac cggacaaaaa   120
ggatccccaa ttctcatgtt tgacagctta tcatcgataa gctttaatgc ggtagtttat   180
cacagttaaa ttgctaacgc agtcaggcac cgtgtatgaa atctaacaat gcgctcatcg   240
tcatcctcgg caccgtcacc ctggatgctg taggcatagg cttggttatg ccggtactgc   300
cgggcctctt gcgggatatc gtccattccg acagcatcgc cagtcactat ggcgtgctgc   360
tagcgctata tgcgttgatg caatttctat gcgcacccgt tctcggagca ctgtccgacc   420
gctttggccg ccgcccagtc ctgctcgctt cgctacttgg agccactatc gactacgcga   480
tcatggcgac cacacccgtc ctgtggatcc tctacgccgg acgcatcgtg gccggcatca   540
ccggcgccac aggtgcggtt gctggcgcct atatcgccga catcaccgat ggggaagatc   600
gggctcgcca cttcgggctc atgagcgctt gtttcggcgt gggtatggtg gcaggccccg   660
tggccggggg actgttgggc gccatctcct tgcatgcacc attccttgcg gcggcggtgc   720
tcaacggcct caacctacta ctgggctgct tcctaatgca ggagtcgcat aagggagagc   780
gtcgaccgat gcccttgaga gccttcaacc cagtcagctc cttccggtgg gcgcggggca   840
tgactatcgt cgccgcactt atgactgtct tctttatcat gcaactcgta ggacaggtgc   900
cggcagcgct ctgggtcatt ttcggcgagg accgctttcg ctggagcgcg acgatgatcg   960
gcctgtcgct tgcggtattc ggaatcttgc acgccctcgc tcaagccttc gtcactggtc   1020
ccgccaccaa acgtttcggc gagaagcagg ccattatcgc cggcatggcg gccgacgcgc   1080
tgggctacgt cttgctggcg ttcgcgacgc gaggctggat ggccttcccc attatgattc   1140
ttctcgcttc cggcggcatc gggatgcccg cgttgcaggc catgctgtcc aggcaggtag   1200
atgacgacca tcagggacag cttcaaggat cgctcgcggc tcttaccagc ctaacttcga   1260
tcactggacc gctgatcgtc acggcgattt atgccgcctc ggcgagcaca tggaacgggt   1320
tggcatggat tgtaggcgcc gccctatacc ttgtctgcct ccccgcgttg cgtcgcggtg   1380
catggagccg ggccacctcg acctgaggga ccctagaggt cccctttttt attttaaaaa   1440
ttttttcaca aaacggttta caagcataac tagtgcggcc gcaagcttgc atgcctgcag   1500
gtcgactcta gactacaaga atgtcccgcc gattcggctg gcggccaggg ccagcagcca   1560
agcgaaggac agggaataga ccaccgagaa tccggcccac cgccagctct gcgcctcctt   1620
gccgatgatg gcgatggtcg acaggcaagg cgcgtagagc aaggtgaaga tcatgaaggc   1680
gatggccgtg gcgggcggca tggcgcccct caaggtctcg gtcagctggt ccgacccctt   1740
ctcctgggca tagatgacgg cataggtcgc caccaccact tccttggcga caaagccggt   1800
catgatggcc gccgaatccc gccaggaaaa gcccagcggc tggaacagcg gcgtcacchc   1860
gatggccgaa cgccccagat agctcttttc cagcttctcc tgcgcctggg cgcttttcag   1920
cgtcttcagc gccgtttcct tttccgggct gtcgggaagc gtggtctgtt cggcgatgac   1980
cgtctcgaaa tcctgggaat aggtgatatc gcgggggaac tcctggagga accagatcac   2040
gatggagccc accaggatga cgccggttac cttggcgaca aagccctggg cgctgtccca   2100
catatggtgc agcaccgaat gcagggtcgg caggcgatag ggcggcagtt caatcacgaa   2160
ggcgtcgcca ccgctgccgg ggatgatgcg gttcagcaac accgccgaca ccatggcggc   2220
gaccagggac agcatataca tggcgaagac cacggtgccg gcgatatcgg cgaagaaagc   2280
tccggcgaac aggataaaga ccggcagtcg ggccgagcag ttcatgaacg gcgcgatcag   2340
catggcgatc agacgcgccc gcttattggt gatgacgcgg gtggccatga cggcggggac   2400
attgcagccg aagccggcca ccatggggat cagggtagtg ccgtgcaggc cgaaatgatg   2460
catcacccgg tccatcagga agctggcgcg ggccagatag ccggtgccgc tgaggatggc   2520
catgaagaag aacagaatga cgatattggg caggaacacg atggttccgc cgacgccggc   2580
catgacgccg ttgaccacga gatcgtggaa catgccctcg ggcagggtct tgtccaccag   2640
atcggtggca atctgaacca aagctttgat ccagtcggtg ggaatggcac ccaggctgaa   2700
agtcgtctcg aacatcaccc acaaaatgcc cagcagcagg ggcaggccca aggtgcggtt   2760
caggaagaaa tcgtcgagga tgcgggtcag cttgaatccg gcggtggggt cgccctcctg   2820
ctggcgggct tcgcgcagca ggccgttggc aaaggcgaac ctcgccgagg ccagcaggcc   2880
ggcggtgtcc tcatcatgct cgtgggccag ggctgtctgc tcggccttga tctcctcgac   2940
cagttcgaca tggttgcttt cgtggcgcag aacctcgtca tcgccttcca gcatcttgat   3000
cgccagccag cgcgagcgca ccgggctcag ctcctggggg tgcagcttgg acagatgctg   3060
ctggacgcgc tcgatggccg cctccagatg gctgtcatag tgtagccgca acggggcggc   3120
ggcgggggtt gccgaggcca tggccgccac cgcgtccagc agggcatcga tgccctcctt   3180
cttcagggcg caggtctcca ccaccggcga tccgagggcc gaggccaata gggcggtgtc   3240
gatgcgaata ccggcccgcc ggacctcgtc catcatgttc aacacggtga tgcggggcag   3300
cccggtctcg atcagctggg tggtcaggaa caggctgcgg tccagatgac cggcatccag   3360
cacattgacg atgatgtcgg gttcctcgcc gtgaatatag tcgcagccga ccttctcctc   3420
cggcgattgc gacgaggtgg aaaaaatgcc cggcacatcg acgatgcgta agggaacccc   3480
gccatgggtg atctcgcgca cattcaggga ggttgtaacc cgctgatagt tggcgacgga   3540
ggattgggcg cctgtcaggg cattgaacaa gctggtggaa ccggcattgg ggttacccac   3600

cagtgccacg gtgacggggg gtttcatata cggctaccct tgaagcgatg tcggatgggg 3660
tgattatttc aagcgaatgc ggatctgccg ggcctcggca ttgcgcaggc ccaggctata 3720
gcccatcagg ctgtagatgc gcggatctcc gagcggagca gagcgatcca gggttacagc 3780
agtcccggac accactccaa gcgactgcat acgacgcttg aaaacgccat cctcggtttc 3840
gatcttgacg atctcaccct gctgctcggg acaaaggtcg gcaagggtaa gagtgtcgat 3900
cttgacctgc tgatccatgg tacccacaat tcacctccaa ttcgcacgga ctatagcaac 3960
gaatcgaggt cggttgacaa gccataaaaa ctgggttcat acgaaggaac ctcgcaatgt 4020
ggcacactta gcgcgctagc acatttcgcg catctgctcg aaacaagacg tcacatcttt 4080
gcgccatcgc ctaccgatca ggatgccgaa ttcgcgcggc cgcggcctag gcggccttaa 4140
ttaaagatgt gtataagaga cag 4163